# EUROPÄISCHE PATENTSCHRIFT

(11) **EP 3 660 146 B1**
(45) Veröffentlichungstag und Bekanntmachung des Hinweises auf die Patenterteilung: **15.11.2023**
(21) Anmeldenummer: 18209177.7
(22) Anmeldetag: 29.11.2018
(51) Int. Cl.: C12N 9/00, C12N 9/54, C12N 15/10, C11D 3/386

(54) **LEISTUNGSVERBESSERTE UND LAGERSTABILE PROTEASE-VARIANTEN**
STORAGE STABLE PROTEASE VARIANTS WITH IMPROVED PERFORMANCE
VARIANTES DE PROTHÈSE STABLES AU STOCKAGE ET À LA PERFORMANCE AMÉLIORÉE

(43) Veröffentlichungstag der Anmeldung: 03.06.2020
(73) Patentinhaber: Henkel AG & Co. KGaA, 40589 Düsseldorf (DE)
(72) Erfinder: Degering, Christian, 40699 Erkrath (DE); Fernandez, Layla, 50858 Köln (DE); Griemert, Sabine, 40789 Monheim am Rhein (DE); Mussmann, Nina, 47877 Willich (DE); Prueser, Inken, 40215 Düsseldorf (DE); Strauss, Daria, 40627 Düsseldorf (DE); Wieland, Susanne, 41541 Zons/Dormagen (DE)

(56) Entgegenhaltungen:
- WO-A1-95/23221
- WO-A1-2018/069158
- DE-A1-102004 027 091

## Beschreibung

Die Erfindung liegt auf dem Gebiet der Enzymtechnologie. Die Erfindung betrifft Proteasen, deren Aminosäuresequenz insbesondere im Hinblick auf den Einsatz in Wasch- und Reinigungsmitteln, insbesondere im Hinblick auf flüssige Wasch- und Reinigungsmittel, verändert wurden, um ihnen eine bessere Lagerstabilität zu verleihen und/oder um ihre Reinigungsleistung zu verbessern, und die für sie codierende Nukleinsäuren sowie deren Herstellung. Die Erfindung betrifft ferner die Verwendungen dieser Proteasen und Verfahren, in denen sie eingesetzt werden, sowie diese enthaltende Mittel, insbesondere Wasch- und Reinigungsmittel, insbesondere flüssige Wasch- und Reinigungsmittel.

Proteasen gehören zu den technisch bedeutendsten Enzymen überhaupt. Für Wasch- und Reinigungsmittel sind sie die am längsten etablierten und in praktisch allen modernen, leistungsfähigen Wasch- und Reinigungsmitteln enthaltenen Enzyme. Sie bewirken den Abbau proteinhaltiger Anschmutzungen auf dem Reinigungsgut. Hierunter sind wiederum Proteasen vom Subtilisin-Typ (Subtilasen, Subtilopeptidasen, EC 3.4.21.62) besonders wichtig, welche aufgrund der katalytisch wirksamen Aminosäuren Serin-Proteasen sind. Sie wirken als unspezifische Endopeptidasen und hydrolysieren beliebige Säureamidbindungen, die im Inneren von Peptiden oder Proteinen liegen. Ihr pH-Optimum liegt meist im deutlich alkalischen Bereich. Einen Überblick über diese Familie bietet beispielsweise der Artikel "Subtilases: Subtilisin-like Proteases" von R. Siezen, Seiten 75-95 in "Subtilisin enzymes", herausgegeben von R. Bott und C. Betzel, New York, 1996. Subtilasen werden natürlicherweise von Mikroorganismen gebildet. Hierunter sind insbesondere die von Bacillus-Spezies gebildeten und sezernierten Subtilisine als bedeutendste Gruppe innerhalb der Subtilasen zu erwähnen.

Beispiele für die in Wasch- und Reinigungsmitteln bevorzugt eingesetzten Proteasen vom Subtilisin-Typ sind die Subtilisine BPN' und Carlsberg, die Protease PB92, die Subtilisine 147 und 309, die alkalische Protease aus *Bacillus lentus,* insbesondere aus *Bacillus lentus* DSM 5483, Subtilisin DY und die den Subtilasen, nicht mehr jedoch den Subtilisinen im engeren Sinne zuzuordnenden Enzyme Thermitase, Proteinase K und die Proteasen TW3 und TW7, sowie Varianten der genannten Proteasen, die eine gegenüber der Ausgangsprotease veränderte Aminosäuresequenz aufweisen. Proteasen werden durch aus dem Stand der Technik bekannte Verfahren gezielt oder zufallsbasiert verändert und so beispielsweise für den Einsatz in Wasch- und Reinigungsmitteln optimiert. Dazu gehören Punkt-, Deletions- oder Insertionsmutagenese oder Fusion mit anderen Proteinen oder Proteinteilen. So sind für die meisten aus dem Stand der Technik bekannten Proteasen entsprechend optimierte Varianten bekannt.

In den internationalen Patentanmeldungen WO95/23221A1, WO92/21760A1, WO 2018/069158 und WO2013/060621A1 sowie in der deutschen Patentanmeldung DE102004027091 sind Varianten der alkalischen Protease aus *Bacillus lentus* DSM 5483 offenbart, die für ihren Einsatz in Wasch- oder Reinigungsmitteln geeignet sind. Ferner sind in der internationalen Patentanmeldung WO2011/032988A1 und der europäischen Patentanmeldung EP3044302A1 Wasch- und Reinigungsmittel offenbart, die Varianten der alkalischen Protease aus *Bacillus lentus* DSM 5483 enthalten. Die in diesen Schriften offenbarten Protease-Varianten können neben weiteren Positionen an den Positionen 3, 4, 99 und/oder 199 in der Zählweise der alkalischen Protease aus *Bacillus lentus* DSM 5483 verändert sein und beispielsweise an den besagten Positionen die Aminosäuren 3T, 4I, 99E oder 1991 aufweisen. Kombinationen weiterer Veränderungen, wie sie nachstehend beschrieben werden, gehen aus diesen Schriften aber nicht hervor.

Generell sind nur ausgewählte Proteasen für den Einsatz in flüssigen Tensid-haltigen Zubereitungen überhaupt geeignet. Viele Proteasen zeigen in derartigen Zubereitungen keine ausreichende katalytische Leistung. Für die Anwendung von Proteasen in Wasch- und Reinigungsmitteln ist daher eine hohe katalytische Aktivität unter Bedingungen wie sie sich während eines Waschgangs darstellen und eine hohe Lagerstabilität besonders wünschenswert.

Folglich haben Protease- und Tensid-haltige flüssige Formulierungen aus dem Stand der Technik den Nachteil, dass die enthaltenen Proteasen unter Standard-Waschbedingungen (z.B. in einem Temperaturbereich von 20°C bis 40°C) keine zufriedenstellende proteolytische Aktivität aufweisen oder nicht ausreichend lagerstabil sind, und die Formulierungen daher keine optimale Reinigungsleistung an proteasesensitiven Anschmutzungen zeigen.

Überraschenderweise wurde jetzt festgestellt, dass eine Protease vom Typ der alkalischen Protease aus *Bacillus lentus* DSM 5483 oder eine hierzu hinreichend ähnliche Protease (bezogen auf die Sequenzidentität), die bezogen auf die Nummerierung gemäß SEQ ID NO:1 an (i) den Positionen, die den Positionen 3, 4, 99 und 199 entsprechen, die Aminosäuresubstitutionen 3T, 4I, 99E und 1991, aufweist, und (ii) mindestens einer der Positionen, die den Positionen 9, 21, 42, 44, 105, 112, 113, 131, 137, 139, 141, 145, 159, 168, 176, 177, 182, 193, 198, 204, 205, 206, 210, 212, 230, 234, 250, 253, 255, 259 oder 267 entsprechen, mindestens eine Aminosäuresubstitution, insbesondere mindestens eine Aminosäuresubstitution, die aus der aus 9C, 21F, 21W, 42S, 42H, 44S, 105V, 112V, 113A, 131D, 1371, 139R, 141S, 1451, 159L, 168V, 176E, 177D, 182C, 193M, 198D, 204L, 205D, 206A, 210C, 212D, 230E, 234A, 250N, 253C, 255Y, 259C und 267S bestehenden Gruppe ausgewählt ist, aufweist, hinsichtlich ihrer Lagerstabilität und/oder ihrer Waschleistung und/oder ihrer Stabilität gegenüber der Wildtypform (SEQ ID NO:1) bzw. einer Ausgangsvariante, wie in WO2013060621A1 beschrieben, verbessert ist und daher besonders für den Einsatz in Wasch- oder Reinigungsmitteln geeignet ist.

Gegenstand der Erfindung ist daher eine Protease, umfassend eine Aminosäuresequenz, die mindestens 70% Sequenzidentität mit der in SEQ ID NO:1 angegebenen Aminosäuresequenz über deren Gesamtlänge aufweist und bezogen auf die Nummerierung gemäß SEQ ID NO:1 an (i) den Positionen, die den Positionen 3, 4, 99 und 199 entsprechen, mindestens die Aminosäuresubstitutionen S3T, V4I, R99E und V199I aufweist, und (ii) mindestens einer der Positionen, die den Positionen 9, 21, 42, 44, 105, 112, 113, 131, 137, 139, 141, 145, 159, 168, 176, 177, 182, 193, 198, 204, 205, 206, 210, 212, 230, 234, 250, 253, 255, 259 oder 267 entsprechen, mindestens eine Aminosäuresubstitution aufweist.

Ein weiterer Gegenstand der Erfindung ist ein Verfahren zur Herstellung einer wie oben definierten Protease umfassend das Einbringen von Aminosäuresubstitutionen S3T, V4I, R99E und V199I an (i) den Positionen, die bezogen auf die Nummerierung gemäß SEQ ID NO:1 den Positionen 3, 4, 99 und 199 entsprechen, und (ii) mindestens einer der Positionen, die bezogen auf die Nummerierung gemäß SEQ ID NO:1 den Positionen 9, 21, 42, 44, 105, 112, 113, 131, 137, 139, 141, 145, 159, 168, 176, 177, 182, 193, 198, 204, 205, 206, 210, 212, 230, 234, 250, 253, 255, 259 oder 267 entsprechen, in ein Ausgangsmolekül, das eine Aminosäuresequenz aufweist, die mindestens 70% Sequenzidentität mit der in SEQ ID NO:1 angegebenen Aminosäuresequenz über deren Gesamtlänge aufweist.

Eine Protease im Sinne der vorliegenden Patentanmeldung umfasst daher sowohl die Protease als solche als auch eine mit einem erfindungsgemäßen Verfahren hergestellte Protease. Alle Ausführungen zur Protease beziehen sich daher sowohl auf die Protease als solche wie auch auf die mittels entsprechender Verfahren hergestellten Proteasen.

Weitere Aspekte der Erfindung betreffen die für diese Proteasen codierenden Nukleinsäuren, erfindungsgemäße Proteasen oder Nukleinsäuren enthaltende nicht-menschliche Wirtszellen sowie erfindungsgemäße Proteasen umfassende Mittel, insbesondere Wasch- und Reinigungsmittel, Wasch- und Reinigungsverfahren, und Verwendungen der erfindungsgemäßen Proteasen in Wasch- oder Reinigungsmitteln zur Entfernung von proteinhaltigen Anschmutzungen.

Diese und weitere Aspekte, Merkmale und Vorteile der Erfindung werden für den Fachmann aus dem Studium der folgenden detaillierten Beschreibung und Ansprüche ersichtlich. Dabei kann jedes Merkmal aus einem Aspekt der Erfindung in jedem anderen Aspekt der Erfindung eingesetzt werden. Ferner ist es selbstverständlich, dass die hierin enthaltenen Beispiele die Erfindung beschreiben und veranschaulichen sollen, diese aber nicht einschränken und insbesondere die Erfindung nicht auf diese Beispiele beschränkt ist.

Numerische Bereiche, die in dem Format "von x bis y" angegeben sind, schließen die genannten Werte ein. Wenn mehrere bevorzugte numerische Bereiche in diesem Format angegeben sind, ist es selbstverständlich, dass alle Bereiche, die durch die Kombination der verschiedenen Endpunkte entstehen, ebenfalls erfasst werden.

"Mindestens eine", wie hierin verwendet, bedeutet eine oder mehrere, d.h. 1, 2, 3, 4, 5, 6, 7, 8, 9, 10, 11, 12, 13, 14 oder mehr.

"Flüssig", wie hierin verwendet, schließt Flüssigkeiten und Gele sowie auch pastöse Zusammensetzungen ein. Es ist bevorzugt, dass die flüssigen Zusammensetzungen bei Raumtemperatur fließfähig und gießbar sind, es ist aber auch möglich, dass sie eine Fließgrenze aufweisen.

Die vorliegende Erfindung basiert auf der überraschenden Erkenntnis der Erfinder, dass Aminosäuresubstitutionen an den hierin beschriebenen Positionen eine verbesserte Lagerstabilität und/oder eine verbesserte Reinigungsleistung dieser veränderten Protease in Wasch- und Reinigungsmitteln bewirkt.

In bevorzugten Ausführungsformen weist die erfindungsgemäße Protease an (i) den Positionen, die den Positionen 3, 4, 99 und 199 entsprechen, die Aminosäuresubstitutionen auf, die aus der aus 3T, 4I, 99E und 199I bestehenden Gruppe ausgewählt sind, und (ii) mindestens einer der Positionen, die den Positionen 9, 21, 42, 44, 105, 112, 113, 131, 137, 139, 141, 145, 159, 168, 176, 177, 182, 193, 198, 204, 205, 206, 210, 212, 230, 234, 250, 253, 255, 259 oder 267 entsprechen, mindestens eine Aminosäuresubstitution auf, die aus der aus 9C, 21F, 21W, 42S, 42H, 44S, 105V, 112V, 113A, 131D, 137I, 139R, 141S, 145I, 159L, 168V, 176E, 177D, 182C, 193M, 198D, 204L, 205D, 206A, 210C, 212D, 230E, 234A, 250N, 253C, 255Y, 259C oder267S bestehenden Gruppe ausgewählt ist, wobei die Kombination der Aminosäuresubstitutionen aus Gruppe (i) und der mindestens einen Aminosäuresubstitution aus Gruppe (ii) zu einer verbesserten Reinigungsleistung dieser veränderten Protease in Wasch- und Reinigungsmitteln an mindestens einer proteasesensitiven Anschmutzung führt.

In bevorzugten Ausführungsformen weist die erfindungsgemäße Protease an (i) den Positionen, die den Positionen 3, 4, 99 und 199 entsprechen, die Aminosäuresubstitutionen auf, die aus der aus 3T, 4I, 99E und 199I bestehenden Gruppe ausgewählt sind, und (ii) mindestens einer der Positionen, die den Positionen 9, 21, 42, 44, 105, 112, 113, 131, 137, 139, 141, 145, 159, 168, 176, 177, 182, 193, 198, 204, 205, 206, 210, 212, 230, 234, 250, 253, 255, 259 oder 267 entsprechen, mindestens eine Aminosäuresubstitution auf, die aus der aus 9C, 21F, 21W, 42S, 42H, 44S, 105V, 112V, 113A, 131D, 137I, 139R, 141S, 145I, 159L, 168V, 176E, 177D, 182C, 193M, 198D, 204L, 205D, 206A, 210C, 212D, 230E, 234A, 250N, 253C, 255Y, 259C oder267S bestehenden Gruppe ausgewählt ist, wobei die Kombination der Aminosäuresubstitutionen aus Gruppe (i) und der mindestens einen Aminosäuresubstitution aus Gruppe (ii) zu einer verbesserten Lagerstabilität dieser veränderten Protease in Wasch- und Reinigungsmitteln führt.

In bevorzugten Ausführungsformen weist die erfindungsgemäße Protease an (i) den Positionen, die den Positionen 3, 4, 99 und 199 entsprechen, die Aminosäuresubstitutionen auf, die aus der aus 3T, 4I, 99E und 199I bestehenden Gruppe ausgewählt sind, und (ii) mindestens einer der Positionen, die den Positionen 9, 21, 42, 44, 105, 112, 113, 131, 137, 139, 141, 145, 159, 168, 176, 177, 182, 193, 198, 204, 205, 206, 210, 212, 230, 234, 250, 253, 255, 259 oder 267 entsprechen, mindestens eine Aminosäuresubstitution auf, die aus der aus 9C, 21F, 21W, 42S, 42H, 44S, 105V, 112V, 113A, 131D, 137I, 139R, 141S, 145I, 159L, 168V, 176E, 177D, 182C, 193M, 198D, 204L, 205D, 206A, 210C, 212D, 230E, 234A, 250N, 253C, 255Y, 259C oder267S bestehenden Gruppe ausgewählt ist, wobei die Kombination der Aminosäuresubstitutionen aus Gruppe (i) und der mindestens einen Aminosäuresubstitution aus Gruppe (ii) sowohl zu einer verbesserten Reinigungsleistung als auch zu einer verbesserten Lagerstabilität dieser veränderten Protease in Wasch- und Reinigungsmitteln führt.

Bestimmte Ausführungsformen der erfindungsgemäßen Proteasen verfügen über eine verbesserte Lagerstabilität. Sie verfügen über eine erhöhte Stabilität in Wasch- oder Reinigungsmitteln im Vergleich zu dem Wildtypenzym (SEQ ID NO:1) sowie insbesondere auch gegenüber der Ausgangsvariante der Protease (SEQ ID NO:2 aus WO2013/060621A1), insbesondere bei einer Lagerung von 3 oder mehr Tagen, 4 oder mehr Tagen, 7 oder mehr Tagen, 10 oder mehr Tagen, 12 oder mehr Tagen, 14 oder mehr Tagen, 21 oder mehr Tagen oder 28 oder mehr Tagen.

Bestimmte Ausführungsformen der erfindungsgemäßen Proteasen können unabhängig von oder zusätzlich zu der erhöhten Lagerstabilität über eine erhöhte katalytische Aktivität in Wasch- oder Reinigungsmitteln verfügen. In vielfältigen Ausführungsformen können die erfindungsgemäßen Proteasen eine proteolytische Aktivität besitzen, die bezogen auf den Wildtyp (SEQ ID NO:1) und/oder eine bereits leistungsverbesserte Ausgangsvariante der Protease (SEQ ID NO:2 aus WO2013/060621A1) mindestens 101%, 102%, 103%, 104%, 105%, 106%, 107%, 108%, 109% oder 110% beträgt. Solche leistungsverbesserten Proteasen ermöglichen verbesserte Waschergebnisse an proteolytisch-sensitiven Anschmutzungen in verschiedenen Temperaturbereichen, insbesondere einem Temperaturbereich von 20°C bis 40°C.

Ferner verfügen bevorzugte Ausführungsformen erfindungsgemäßer Proteasen über eine besondere Stabilität in Wasch- oder Reinigungsmitteln, beispielsweise gegenüber Tensiden und/oder Bleichmitteln und/oder Chelatoren, und/oder gegenüber Temperatureinflüssen, insbesondere gegenüber hohen Temperaturen, beispielsweise zwischen 50°C und 65°C, insbesondere 60°C, und/oder gegenüber sauren oder alkalischen Bedingungen und/oder gegenüber pH-Wert-Änderungen und/oder gegenüber denaturierenden oder oxidierenden Agentien und/oder gegenüber proteolytischem Abbau und/oder gegenüber einer Veränderung der Redox-Verhältnisse. Mit besonders bevorzugten Ausführungsformen der Erfindung werden folglich leistungsverbesserte und/oder temperaturstabilere Protease-Varianten bereitgestellt. Mit weiteren ganz besonders bevorzugten Ausführungsformen der Erfindung werden leistungsverbesserte und temperaturstabilere Protease-Varianten bereitgestellt. Solche vorteilhaften Ausführungsformen erfindungsgemäßer Proteasen ermöglichen folglich verbesserte Waschergebnisse an proteasesensitiven Anschmutzungen in einem weiten Temperaturbereich.

Unter Reinigungsleistung wird im Rahmen der Erfindung die Aufhellungsleistung an einer oder mehreren Anschmutzungen, insbesondere auf Wäsche oder Geschirr, verstanden. Im Rahmen der Erfindung weist sowohl das Wasch- oder Reinigungsmittel, welches die Protease umfasst bzw. die durch dieses Mittel gebildete Wasch- oder Reinigungsflotte, als auch die Protease selbst eine jeweilige Reinigungsleistung auf. Die Reinigungsleistung des Enzyms trägt somit zur Reinigungsleistung des Mittels bzw. der durch das Mittel gebildeten Wasch- oder Reinigungsflotte bei. Die Reinigungsleistung wird bevorzugt ermittelt wie weiter unten angegeben.

Unter Waschflotte wird diejenige das Wasch- oder Reinigungsmittel enthaltende Gebrauchslösung verstanden, die auf Textilien oder Gewebe bzw. harte Oberflächen einwirkt und damit mit den auf Textilien bzw. Geweben oder harten Oberflächen vorhandenen Anschmutzungen in Kontakt kommt. Üblicherweise entsteht die Waschflotte, wenn der Wasch- oder Reinigungsvorgang beginnt und das Wasch- oder Reinigungsmittel beispielsweise in einer Geschirrspülmaschine, einer Waschmaschine oder in einem anderen geeigneten Behältnis mit Wasser verdünnt wird.

Die erfindungsgemäßen Proteasen weisen enzymatische Aktivität auf, d.h. sie sind zur Hydrolyse von Peptiden und Proteinen befähigt, insbesondere in einem Wasch- oder Reinigungsmittel. Eine erfindungsgemäße Protease ist daher ein Enzym, welches die Hydrolyse von Amid/Peptidbindungen in Protein/Peptid-Substraten katalysiert und dadurch in der Lage ist, Proteine oder Peptide zu spalten. Ferner handelt es sich bei einer erfindungsgemäßen Protease vorzugsweise um eine reife (mature) Protease, d.h. um das katalytisch aktive Molekül ohne Signal- und/oder Propeptid(e). Soweit nicht anders angegeben beziehen sich auch die angegebenen Sequenzen auf jeweils reife (prozessierte) Enzyme.

In verschiedenen Ausführungsformen der Erfindung ist die Protease ein frei vorliegendes Enzym. Dies bedeutet, dass die Protease mit allen Komponenten eines Mittels direkt agieren kann und, falls es sich bei dem Mittel um ein Flüssigmittel handelt, dass die Protease direkt mit dem Lösungsmittel des Mittels (z.B. Wasser) in Kontakt steht. In anderen Ausführungsformen kann ein Mittel Proteasen enthalten, die einen Interaktionskomplex mit anderen Molekülen bilden oder die eine "Umhüllung" enthalten. Hierbei kann ein einzelnes oder mehrere Protease Moleküle durch eine sie umgebende Struktur von den anderen Bestandteilen des Mittels getrennt sein. Eine solche trennende Struktur kann entstehen durch, ist allerdings nicht beschränkt auf, Vesikel, wie etwa eine Micelle oder ein Liposom. Die umgebende Struktur kann aber auch ein Viruspartikel, eine bakterielle Zelle oder eine eukaryotische Zelle sein. In verschiedenen Ausführungsformen kann ein Mittel Zellen von *Bacillus pumilus* oder *Bacillus subtilus,* die die erfindungsgemäßen Proteasen exprimieren, oder Zellkulturüberstände solcher Zellen enthalten.

In besonders bevorzugten Ausführungsformen der Erfindung umfasst die Protease eine Aminosäuresequenz, die zu der in SEQ ID NO:1 angegebenen Aminosäuresequenz über deren Gesamtlänge zu mindestens 70%, 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 98,8%, 99% und 99,5% identisch ist, und bezogen auf die Nummerierung gemäß SEQ ID NO:1 (i) die Aminosäuresubstitutionen 3T, 4I, 99E und 199I aufweist, und (ii) mindestens eine Aminosäuresubstitution an mindestens einer der Positionen, die den Positionen 9, 21, 42, 44, 105, 112, 113, 131, 137, 139, 141, 145, 159, 168, 176, 177, 182, 193, 198, 204, 205, 206, 210, 212, 230, 234, 250, 253, 255, 259 oder 267 entsprechen, aufweist, wobei die mindestens eine Aminosäuresubstitution vorzugsweise aus der aus 9C, 21F, 21W, 42S, 42H, 44S, 105V, 112V, 113A, 131D, 137I, 139R, 141S, 145I, 159L, 168V, 176E, 177D, 182C, 193M, 198D, 204L, 205D, 206A, 210C, 212D, 230E, 234A, 250N, 253C, 255Y, 259C und 267S bestehenden Gruppe ausgewählt ist.

Im Zusammenhang mit der vorliegenden Erfindung bedeutet das Merkmal, dass eine Protease mindestens eine der angegebenen Aminosäuresubstitutionen aufweist, dass sie an der jeweiligen Position eine (der angegebenen) Aminosäuresubstitution(en) enthält, d.h. zumindest die angegebenen Positionen nicht anderweitig mutiert oder, beispielsweise durch Fragmentierung der Protease, deletiert sind.

Die Bestimmung der Identität von Nukleinsäure- oder Aminosäuresequenzen erfolgt durch einen Sequenzvergleich. Dieser Sequenzvergleich basiert auf dem im Stand der Technik etablierten und üblicherweise genutzten BLAST-Algorithmus (vgl. z.B. Altschul et al. (1990) "Basic local alignment search tool", J. Mol. Biol. 215:403-410, und Altschul et al. (1997): "Gapped BLAST and PSI-BLAST: a new generation of protein database search programs", Nucleic Acids Res. 25:3389-3402) und geschieht prinzipiell dadurch, dass ähnliche Abfolgen von Nukleotiden oder Aminosäuren in den Nukleinsäure- oder Aminosäuresequenzen einander zugeordnet werden. Eine tabellarische Zuordnung der betreffenden Positionen wird als Alignment bezeichnet. Ein weiterer im Stand der Technik verfügbarer Algorithmus ist der FASTA-Algorithmus. Sequenzvergleiche (Alignments), insbesondere multiple Sequenzvergleiche, werden mit Computerprogrammen erstellt. Häufig genutzt werden beispielsweise die Clustal-Serie (vgl. z.B. Chenna et al. (2003) "Multiple sequence alignment with the Clustal series of programs", Nucleic Acids Res. 31:3497-3500), T-Coffee (vgl. Z.B. Notredame et al. (2000) "T-Coffee: A novel method for multiple sequence alignments", J. Mol. Biol. 302:205-217) oder Programme, die auf diesen Programmen beziehungsweise Algorithmen basieren. Ferner möglich sind Sequenzvergleiche (Alignments) mit dem Computer-Programm Vector NTI^{®} Suite 10.3 (Invitrogen Corporation, 1600 Faraday Avenue, Carlsbad, Kalifornien, USA) mit den vorgegebenen Standardparametern, dessen AlignX-Modul für die Sequenzvergleiche auf ClustalW basiert. Soweit nicht anders angegeben, wird die hierin angegebene Sequenzidentität mit dem BLAST-Algorithmus bestimmt.

Solch ein Vergleich erlaubt auch eine Aussage über die Ähnlichkeit der verglichenen Sequenzen zueinander. Sie wird üblicherweise in Prozent Identität, das heißt dem Anteil der identischen Nukleotide oder Aminosäurereste an denselben oder in einem Alignment einander entsprechenden Positionen angegeben. Der weiter gefasste Begriff der Homologie bezieht bei Aminosäuresequenzen konservierte Aminosäureaustausche in die Betrachtung mit ein, also Aminosäuren mit ähnlicher chemischer Aktivität, da diese innerhalb des Proteins meist ähnliche chemische Aktivitäten ausüben. Daher kann die Ähnlichkeit der verglichenen Sequenzen auch Prozent Homologie oder Prozent Ähnlichkeit angegeben sein. Identitäts- und/oder Homologieangaben können über ganze Polypeptide oder Gene oder nur über einzelne Bereiche getroffen werden. Homologe oder identische Bereiche von verschiedenen Nukleinsäure- oder Aminosäuresequenzen sind daher durch Übereinstimmungen in den Sequenzen definiert. Solche Bereiche weisen oftmals identische Funktionen auf. Sie können klein sein und nur wenige Nukleotide oder Aminosäuren umfassen. Oftmals üben solche kleinen Bereiche für die Gesamtaktivität des Proteins essentielle Funktionen aus. Es kann daher sinnvoll sein, Sequenzübereinstimmungen nur auf einzelne, gegebenenfalls kleine Bereiche zu beziehen. Soweit nicht anders angegeben beziehen sich Identitäts- oder Homologieangaben in der vorliegenden Anmeldung aber auf die Gesamtlänge der jeweils angegebenen Nukleinsäure- oder Aminosäuresäuresequenz.

Im Zusammenhang mit der vorliegenden Erfindung bedeutet die Angabe, dass eine Aminosäureposition einer numerisch bezeichneten Position in SEQ ID NO:1 entspricht daher, dass die entsprechende Position der numerisch bezeichneten Position in SEQ ID NO: 1 in einem wie oben definierten Alignment zugeordnet ist.

In einer weiteren Ausführungsform der Erfindung ist die Protease dadurch gekennzeichnet, dass ihre Reinigungsleistung (nach Lagerung, z.B. über 3 Wochen) gegenüber dem Wildtypenzym (SEQ ID NO:1) oder einer in WO201360621A1 beschriebenen Ausgangsvariante nicht signifikant verringert ist, d.h. mindestens 80% der Referenzwaschleistung besitzt, vorzugsweise mindestens 100%, noch bevorzugter mindestens 110% oder mehr. Die Reinigungsleistung kann in einem Waschsystem bestimmt werden, das ein Waschmittel in einer Dosierung zwischen 4,5 und 7,0 Gramm pro Liter Waschflotte sowie die Protease enthält, wobei die zu vergleichenden Proteasen konzentrationsgleich (bezogen auf aktives Protein) eingesetzt sind und die Reinigungsleistung gegenüber einer Anschmutzung auf Baumwolle bestimmt wird durch Messung des Reinigungsgrades der gewaschenen Textilien. Beispielsweise kann der Waschvorgang für 60 Minuten bei einer Temperatur von 40°C erfolgen und das Wasser eine Wasserhärte zwischen 15,5 und 16,5° (deutsche Härte) aufweisen. Die Konzentration der Protease in dem für dieses Waschsystem bestimmten Waschmittel beträgt 0,001 bis 0,1 Gew.-%, vorzugsweise 0,01 bis 0,06 Gew.-%, bezogen auf aktives, gereinigtes Protein.

Ein flüssiges Referenzwaschmittel für ein solches Waschsystem kann zum Beispiel wie folgt zusammengesetzt sein (alle Angaben in Gewichts-%): 4,4% Alkylbenzolsulfonsäure, 5,6% weitere anionische Tenside, 2,4% C₁₂-C₁₈ Na-Salze von Fettsäuren (Seifen), 4,4% nicht-ionische Tenside, 0,2% Phosphonate, 1,4% Zitronensäure, 0,95% NaOH, 0,01% Entschäumer, 2% Glycerin, 0,08% Konservierungsstoffe, 1% Ethanol, Rest demineralisiertes Wasser. Bevorzugt beträgt die Dosierung des flüssigen Waschmittels zwischen 4,5 und 6,0 Gramm pro Liter Waschflotte, beispielsweise 4,7, 4,9 oder 5,9 Gramm pro Liter Waschflotte. Bevorzugt wird gewaschen in einem pH-Wertebereich zwischen pH 7 und pH 10,5, bevorzugt zwischen pH 7,5 und pH 8,5.

Im Rahmen der Erfindung erfolgt die Bestimmung der Reinigungsleistung beispielsweise bei 20°C oder 40°C unter Verwendung eines flüssigen Waschmittels wie z.B. des vorstehend angegebenen, wobei der Waschvorgang vorzugsweise für 60 Minuten bei 600 rpm erfolgt.

Der Weißegrad, d.h. die Aufhellung der Anschmutzungen, als Maß für die Reinigungsleistung wird mit optischen Messverfahren bestimmt, bevorzugt photometrisch. Ein hierfür geeignetes Gerät ist beispielsweise das Spektrometer Minolta CM508d. Üblicherweise werden die für die Messung eingesetzten Geräte zuvor mit einem Weißstandard, bevorzugt einem mitgelieferten Weißstandard, kalibriert.

Ein flüssiges Referenzhandgeschirrspülmittel für ein solches Waschsystem kann zum Beispiel wie folgt zusammengesetzt sein (alle Angaben in Gew.-%): 8-20% Alkylbenzolsulfonsäure, 30-80% demineralisiertes Wasser, 5,4% NaOH (50%), 7,14% Fettalkoholethersulfat, 2,0% NaCl (20%), 0,383% Phosphorsäure (H₃PO₄; 34%/85%), 0,1% Konservierungsmittel, 0,25%Parfüm, 1,0% Farbstoff, 0,04% Bitterstoff.

Durch den aktivitätsgleichen Einsatz der jeweiligen Protease wird sichergestellt, dass auch bei einem etwaigen Auseinanderklaffen des Verhältnisses von Aktivsubstanz zu Gesamtprotein (die Werte der spezifischen Aktivität) die jeweiligen enzymatischen Eigenschaften, also beispielsweise die Reinigungsleistung an bestimmten Anschmutzungen, verglichen werden. Generell gilt, dass eine niedrige spezifische Aktivität durch Zugabe einer größeren Proteinmenge ausgeglichen werden kann.

Ansonsten sind Verfahren zur Bestimmung der Protease-Aktivität dem Fachmann auf dem Gebiet der Enzymtechnologie geläufig und werden von ihm routinemäßig angewendet. Beispielsweise sind solche Verfahren offenbart in Tenside, Band 7 (1970), S. 125-132. Alternativ kann die Protease-Aktivität über die Freisetzung des Chromophors para-Nitroanilin (pNA) aus dem Substrat suc-L-Ala-L-Ala-L-Pro-L-Phe-p-Nitroanilid (AAPF) bestimmt werden. Die Protease spaltet das Substrat und setzt pNA frei. Die Freisetzung des pNA verursacht eine Zunahme der Extinktion bei 410 nm, deren zeitlicher Verlauf ein Maß für die enzymatische Aktivität ist (vgl. Del Mar et al., 1979). Die Messung erfolgt bei einer Temperatur von 25°C, bei pH 8,6, und einer Wellenlänge von 410 nm. Die Messzeit beträgt 5 min und das Messintervall 20s bis 60s. Die Protease-Aktivität wird üblicherweise in Protease-Einheiten (PE) angegeben. Geeignete Protease-Aktivitäten betragen beispielsweise 2,25, 5 oder 10 PE pro ml Waschflotte. Die Protease-Aktivität ist jedoch nicht gleich Null.

Ein alternativer Test zur Feststellung der proteolytischen Aktivität der erfindungsgemäßen Proteasen ist ein optisches Messverfahren, bevorzugt ein photometrisches Verfahren. Der hierfür geeignete Test umfasst die Protease-abhängige Spaltung des Substratproteins Casein. Dieses wird durch die Protease in eine Vielzahl kleinerer Teilprodukte gespalten. Die Gesamtheit dieser Teilprodukte weist eine erhöhte Absorption bei 290 nm gegenüber nicht gespaltenem Casein auf, wobei diese erhöhte Absorption unter Verwendung eines Photometers ermittelt werden und somit ein Rückschluss auf die enzymatische Aktivität der Protease gezogen werden kann.

Die Proteinkonzentration kann mit Hilfe bekannter Methoden, zum Beispiel dem BCA-Verfahren (Bicinchoninsäure; 2,2'-Bichinolyl-4,4'-dicarbonsäure) oder dem Biuret-Verfahren (Gornall et al., J. Biol. Chem. 177 (1948): 751-766) bestimmt werden. Die Bestimmung der Aktivproteinkonzentration kann diesbezüglich über eine Titration der aktiven Zentren unter Verwendung eines geeigneten irreversiblen Inhibitors und Bestimmung der Restaktivität (vgl. Bender et al., J. Am. Chem. Soc. 88, 24 (1966): 5890-5913) erfolgen.

Zusätzlich zu den vorstehend erläuterten Aminosäureveränderungen können erfindungsgemäße Proteasen weitere Aminosäureveränderungen, insbesondere AminosäureSubstitutionen, -Insertionen oder -Deletionen, aufweisen. Solche Proteasen sind beispielsweise durch gezielte genetische Veränderung, d.h. durch Mutageneseverfahren, weiterentwickelt und für bestimmte Einsatzzwecke oder hinsichtlich spezieller Eigenschaften (beispielsweise hinsichtlich ihrer katalytischen Aktivität, Stabilität, usw.) optimiert. Ferner können erfindungsgemäße Nukleinsäuren in Rekombinationsansätze eingebracht und damit zur Erzeugung völlig neuartiger Proteasen oder anderer Polypeptide genutzt werden.

Das Ziel ist es, in die bekannten Moleküle gezielte Mutationen wie Substitutionen, Insertionen oder Deletionen einzuführen, um beispielsweise die Reinigungsleistung von erfindungsgemäßen Enzymen zu verbessern. Hierzu können insbesondere die Oberflächenladungen und/oder der isoelektrische Punkt der Moleküle und dadurch ihre Wechselwirkungen mit dem Substrat verändert werden. So kann beispielsweise die Nettoladung der Enzyme verändert werden, um darüber die Substratbindung insbesondere für den Einsatz in Wasch- und Reinigungsmitteln zu beeinflussen. Alternativ oder ergänzend kann durch eine oder mehrere entsprechende Mutationen die Stabilität oder katalytische Aktivität der Protease erhöht und dadurch ihre Reinigungsleistung verbessert werden. Vorteilhafte Eigenschaften einzelner Mutationen, z.B. einzelner Substitutionen, können sich ergänzen. Eine hinsichtlich bestimmter Eigenschaften bereits optimierter Proteasen, zum Beispiel hinsichtlich ihrer Stabilität bei Lagerung, kann daher im Rahmen der Erfindung zusätzlich weiterentwickelt sein.

Für die Beschreibung von Substitutionen, die genau eine Aminosäureposition betreffen (Aminosäureaustausche), wird hierin folgende Konvention angewendet: zunächst wird die natürlicherweise vorhandene Aminosäure in Form des international gebräuchlichen Einbuchstaben-Codes bezeichnet, dann folgt die zugehörige Sequenzposition und schließlich die eingefügte Aminosäure. Mehrere Austausche innerhalb derselben Polypeptidkette werden durch Schrägstriche voneinander getrennt. Bei Insertionen sind nach der Sequenzposition zusätzliche Aminosäuren benannt. Bei Deletionen ist die fehlende Aminosäure durch ein Symbol, beispielsweise einen Stern oder einen Strich, ersetzt oder vor der entsprechenden Position ein Δ angegeben. Beispielsweise beschreibt A95G die Substitution von Alanin an Position 95 durch Glycin, A95AG die Insertion von Glycin nach der Aminosäure Alanin an Position 95 und A95* oder ΔA59 die Deletion von Alanin an Position 95. Diese Nomenklatur ist dem Fachmann auf dem Gebiet der Enzymtechnologie bekannt.

Ein weiterer Gegenstand der Erfindung ist daher eine Protease, die dadurch gekennzeichnet ist, dass sie aus einer Protease wie vorstehend beschrieben als Ausgangsmolekül erhältlich ist durch ein- oder mehrfache konservative Aminosäuresubstitution, wobei die Protease in der Zählung gemäß SEQ ID NO:1 mindestens eine der vorstehend beschriebenen Aminosäuresubstitutionen aufweist. Der Begriff "konservative Aminosäuresubstitution" bedeutet den Austausch (Substitution) eines Aminosäurerestes gegen einen anderen Aminosäurerest, wobei dieser Austausch nicht zu einer Änderung der Polarität oder Ladung an der Position der ausgetauschten Aminosäure führt, z.B. der Austausch eines unpolaren Aminosäurerestes gegen einen anderen unpolaren Aminosäurerest. Konservative Aminosäuresubstitutionen im Rahmen der Erfindung umfassen beispielsweise: G=A=S, I=V=L=M, D=E, N=Q, K=R, Y=F, S=T, G=A=I=V=L=M=Y=F=W=P=S=T.

Alternativ oder ergänzend ist die Protease dadurch gekennzeichnet, dass sie aus einer erfindungsgemäßen Protease als Ausgangsmolekül erhältlich ist durch Fragmentierung, Deletions-, Insertions- oder Substitutionsmutagenese und eine Aminosäuresequenz umfasst, die über eine Länge von mindestens 190, 200, 210, 220, 230, 240, 250, 260, 261, 262, 263, 264, 265, 266, 267, 268 oder 269 zusammenhängenden Aminosäuren mit dem Ausgangsmolekül übereinstimmt, wobei die Protease (i) Aminosäuresubstitutionen an den Positionen gemäss Anspruch 1, die den Positionen 3, 4, 99 und 199 entsprechen, und (ii) mindestens eine Aminosäuresubstitution an mindestens einer der Positionen, die den Positionen 9, 21, 42, 44, 105, 112, 113, 131, 137, 139, 141, 145, 159, 168, 176, 177, 182, 193, 198, 204, 205, 206, 210, 212, 230, 234, 250, 253, 255, 259 oder 267 entsprechen, aufweist.

So ist es beispielsweise möglich, an den Termini oder in den Loops des Enzyms einzelne Aminosäuren zu deletieren, ohne dass dadurch die proteolytische Aktivität verloren oder vermindert wird. Ferner kann durch derartige Fragmentierung, Deletions-, Insertions- oder Substitutionsmutagenese beispielsweise auch die Allergenizität betreffender Enzyme gesenkt und somit insgesamt ihre Einsetzbarkeit verbessert werden. Vorteilhafterweise behalten die Enzyme auch nach der Mutagenese ihre proteolytische Aktivität, d.h. ihre proteolytische Aktivität entspricht mindestens derjenigen des Ausgangsenzyms, d.h. in einer bevorzugten Ausführungsform beträgt die proteolytische Aktivität mindestens 80%, vorzugsweise mindestens 90% der Aktivität des Ausgangsenzyms. Auch weitere Substitutionen können vorteilhafte Wirkungen zeigen. Sowohl einzelne wie auch mehrere zusammenhängende Aminosäuren können gegen andere Aminosäuren ausgetauscht werden.

Die Aminosäurepositionen werden hierbei durch ein Alignment der Aminosäuresequenz einer erfindungsgemäßen Protease mit der Aminosäuresequenz der Protease aus *Bacillus lentus,* wie sie in SEQ ID NO: 1 angegeben ist, definiert. Weiterhin richtet sich die Zuordnung der Positionen nach dem reifen (maturen) Protein. Diese Zuordnung ist insbesondere auch anzuwenden, wenn die Aminosäuresequenz einer erfindungsgemäßen Protease eine höhere oder niedrigere Zahl von Aminosäureresten umfasst als die Protease aus *Bacillus lentus* gemäß SEQ ID NO:1. Ausgehend von den genannten Positionen in der Aminosäuresequenz der Protease aus *Bacillus lentus* sind die Veränderungspositionen in einer erfindungsgemäßen Protease diejenigen, die eben diesen Positionen in einem Alignment zugeordnet sind.

Vorteilhafte Positionen für Sequenzveränderungen, insbesondere Substitutionen, der Protease aus *Bacillus lentus,* die übertragen auf homologe Positionen der erfindungsgemäßen Proteasen bevorzugt von Bedeutung sind und der Protease vorteilhafte funktionelle Eigenschaften verleihen, sind demnach die Positionen, die in einem Alignment den hierin beschriebenen Positionen entsprechen, d.h. in der Zählung gemäß SEQ ID NO:1. An den genannten Positionen liegen in dem Wildtypmolekül der Protease aus *Bacillus lentus* (SEQ ID NO: 1) folgende Aminosäurereste: S3, V4, S9, L21, N42, R44, R99, I105, A112, G113, A131, V137, S139, T141, V145, I159, A168, Q176, N177, S182, V193, N198, V199, P204, G205, S206, S210, N212, Q230, S234, S250, S253, N255, S259.

Eine weitere Bestätigung der korrekten Zuordnung der zu verändernden Aminosäuren, d.h. insbesondere deren funktionelle Entsprechung, können Vergleichsversuche liefern, wonach die beiden auf der Basis eines Alignments einander zugeordneten Positionen in beiden miteinander verglichenen Proteasen auf die gleiche Weise verändert werden und beobachtet wird, ob bei beiden die enzymatische Aktivität auf gleiche Weise verändert wird. Geht beispielsweise ein Aminosäureaustausch in einer bestimmten Position der Protease aus *Bacillus lentus* gemäß SEQ ID NO: 1 mit einer Veränderung eines enzymatischen Parameters einher, beispielsweise mit der Erhöhung des K_{M}-Wertes, und wird eine entsprechende Veränderung des enzymatischen Parameters, beispielsweise also ebenfalls eine Erhöhung des K_{M}-Wertes, in einer erfindungsgemäßen Proteasevariante beobachtet, deren Aminosäureaustausch durch dieselbe eingeführte Aminosäure erreicht wurde, so ist hierin eine Bestätigung der korrekten Zuordnung zu sehen.

Alle genannten Sachverhalte sind auch auf die erfindungsgemäßen Verfahren zur Herstellung einer Protease anwendbar. Demnach umfasst ein erfindungsgemäßes Verfahren ferner einen oder mehrere der folgenden Verfahrensschritte:
(a) Einbringen einer ein- oder mehrfachen konservativen Aminosäuresubstitution in die Protease, wobei die Protease aufweist:
   i) Aminosäuresubstitutionen S3T, V4I, R99E und V199I an den Positionen, die den Positionen 3, 4, 99 und 199 entsprechen, und
   ii) an mindestens einer der Positionen, die den Positionen 9, 21, 42, 44, 105, 112, 113, 131, 137, 139, 141, 145, 159, 168, 176, 177, 182, 193, 198, 204, 205, 206, 210, 212, 230, 234, 250, 253, 255, 259 oder 267 entsprechen, mindestens eine Aminosäuresubstitution;
(b) Veränderung der Aminosäuresequenz durch Fragmentierung, Deletions-, Insertions- oder Substitutionsmutagenese derart, dass die Protease eine Aminosäuresequenz umfasst, die über eine Länge von mindestens 190, 200, 210, 220, 230, 240, 250, 260, 261, 262, 263, 264, 265, 266, 267, 268 oder 269 zusammenhängenden Aminosäuren mit dem Ausgangsmolekül übereinstimmt, wobei die Protease aufweist:
   i) Aminosäuresubstitutionen S3T, V4I, R99E und V199I an den Positionen, die den Positionen 3, 4, 99 und 199 entsprechen, und
   ii) an mindestens einer der Positionen, die den Positionen 9, 21, 42, 44, 105, 112, 113, 131, 137, 139, 141, 145, 159, 168, 176, 177, 182, 193, 198, 204, 205, 206, 210, 212, 230, 234, 250, 253, 255, 259 oder 267 entsprechen, mindestens eine Aminosäuresubstitution.

Sämtliche Ausführungsformen gelten auch für die erfindungsgemäßen Verfahren.

In weiteren Ausgestaltungen der Erfindung ist die Protease bzw. die mit einem erfindungsgemäßen Verfahren hergestellte Protease noch mindestens zu 70%, 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 98,8%, 99% oder 99,5% identisch zu der in SEQ ID NO:1 angegebenen Aminosäuresequenz über deren Gesamtlänge. Die Protease bzw. die mit einem erfindungsgemäßen Verfahren hergestellte Protease weist (i) die Aminosäuresubstitutionen 3T, 4I, 99E und 199I an den Positionen, die den Positionen 3, 4, 99 und 199 entsprechen, und (ii) mindestens eine der Aminosäuresubstitutionen 9C, 21F, 21W, 42S, 42H, 44S, 105V, 112V, 113A, 131D, 137I, 139R, 141S, 145I, 159L, 168V, 176E, 177D, 182C, 193M, 198D, 204L, 205D, 206A, 210C, 212D, 230E, 234A, 250N, 253C, 255Y, 259C oder 267S an mindestens einer der Positionen, die den Positionen 9, 21, 42, 44, 105, 112, 113, 131, 137, 139, 141, 145, 159, 168, 176, 177, 182, 193, 198, 204, 205, 206, 210, 212, 230, 234, 250, 253, 255, 259 oder 267 entsprechen, jeweils bezogen auf die Nummerierung gemäß SEQ ID NO:1, auf. Beispiele hierfür sind die folgenden Aminosäuresubstitutionsvarianten: (i) S3T + V4I + R99E + V199I + A267S + N255Y; (ii) S3T + V4I + R99E + V199I + N198D + S234A + L21F; (iii) S3T + V4I + R99E + V199I + N212D; (iv) S3T + V4I + R99E + V199I + G205D; (v) S3T + V4I + R99E + V199I + S182C; (vi) S3T + V4I + R99E + V199I + R44S + S234A; (vii) S3T + V4I + R99E + V199I + S253C; (viii) S3T + V4I + R99E + V199I + I105V; (ix) S3T + V4I + R99E + V199I + N177D; (x) S3T + V4I + R99E + V199I + V193M; (xi) S3T + V4I + R99E + V199I + S9C; (xii) S3T + V4I + R99E + V199I + N198D; (xiii) S3T + V4I + R99E + V199I + S259C; (xiv) S3T + V4! + R99E + V199I + I159L + Q230E; (xv) S3T + V4I + R99E + V199I + P204L; (xvi) S3T + V4I + R99E + V199I + L21F; (xvii) S3T + V4! + R99E + V199I + N42H; (xviii) S3T + V4I + R99E + V199I + S139R + S250N; (xix) S3T + V4I + R99E + V199I + N42S; (xx) S3T + V4I + R99E + V199I + V137I; (xxi) S3T + V4I + R99E + V199I + V145I; (xxii) S3T + V4I + R99E + V199I + S206A; (xxiii) S3T + V4I + R99E + V199I + G113A + L21W; (xxiv) S3T + V4I + R99E + V199I + A112V; (xxv) S3T + V4I + R99E + V199I + A168V; (xxvi) S3T + V4I + R99E + V199I + Q176E + A131D; (xxvii) S3T + V4I + R99E + V199I + T141S + S210C, jeweils bezogen auf die Nummerierung gemäß SEQ ID NO: 1, sowie die in den Beispielen beschriebenen Varianten.

Ein weiterer Gegenstand der Erfindung ist eine zuvor beschriebene Protease, die zusätzlich stabilisiert ist, insbesondere durch eine oder mehrere Mutationen, beispielsweise Substitutionen, oder durch Kopplung an ein Polymer. Eine Erhöhung der Stabilität bei der Lagerung und/oder während des Einsatzes, beispielsweise beim Waschprozess, führt dazu, dass die enzymatische Aktivität länger anhält und damit die Reinigungsleistung verbessert wird. Grundsätzlich kommen alle im Stand der Technik beschriebenen und/oder zweckmäßigen Stabilisierungsmöglichkeiten in Betracht. Bevorzugt sind solche Stabilisierungen, die über Mutationen des Enzyms selbst erreicht werden, da solche Stabilisierungen im Anschluss an die Gewinnung des Enzyms keine weiteren Arbeitsschritte erfordern. Beispiele für hierfür geeignete Sequenzveränderungen sind vorstehend genannt. Weitere geeignete Sequenzveränderungen sind aus dem Stand der Technik bekannt.

Weitere Möglichkeiten der Stabilisierung sind beispielsweise:
- Veränderung der Bindung von Metallionen, insbesondere der Calcium-Bindungsstellen, beispielsweise durch Austauschen von einer oder mehreren der an der Calcium-Bindung beteiligten Aminosäure(n) gegen eine oder mehrere negativ geladene Aminosäuren und/oder durch Einführen von Sequenzveränderungen in mindestens einer der Folgen der beiden Aminosäuren Arginin/Glycin;
- Schutz gegen den Einfluss von denaturierenden Agentien wie Tensiden durch Mutationen, die eine Veränderung der Aminosäuresequenz auf oder an der Oberfläche des Proteins bewirken;
- Austausch von Aminosäuren, die nahe dem N-Terminus liegen, gegen solche, die vermutlich über nicht-kovalente Wechselwirkungen mit dem Rest des Moleküls in Kontakt treten und somit einen Beitrag zur Aufrechterhaltung der globulären Struktur leisten.

Bevorzugte Ausführungsformen sind solche, bei denen das Enzym auf mehrere Arten stabilisiert wird, da mehrere stabilisierende Mutationen additiv oder synergistisch wirken.

Ein weiterer Gegenstand der Erfindung ist eine Protease wie vorstehend beschrieben, die dadurch gekennzeichnet ist, dass sie mindestens eine chemische Modifikation aufweist. Eine Protease mit einer solchen Veränderung wird als Derivat bezeichnet, d.h. die Protease ist derivatisiert.

Unter Derivaten werden im Sinne der vorliegenden Anmeldung demnach solche Proteine verstanden, deren reine Aminosäurekette chemisch modifiziert worden ist. Solche Derivatisierungen können beispielsweise *in vivo* durch die Wirtszelle erfolgen, die das Protein exprimiert. Diesbezüglich sind Kopplungen niedrigmolekularer Verbindungen wie von Lipiden oder Oligosacchariden besonders hervorzuheben. Derivatisierungen können aber auch *in vitro* durchgeführt werden, etwa durch die chemische Umwandlung einer Seitenkette einer Aminosäure oder durch kovalente Bindung einer anderen Verbindung an das Protein. Beispielsweise ist die Kopplung von Aminen an Carboxylgruppen eines Enzyms zur Veränderung des isoelektrischen Punkts möglich. Eine solche andere Verbindung kann auch ein weiteres Protein sein, das beispielsweise über bifunktionelle chemische Verbindungen an ein erfindungsgemäßes Protein gebunden wird. Ebenso ist unter Derivatisierung die kovalente Bindung an einen makromolekularen Träger zu verstehen, oder auch ein nichtkovalenter Einschluss in geeignete makromolekulare Käfigstrukturen. Derivatisierungen können beispielsweise die Substratspezifität oder die Bindungsstärke an das Substrat beeinflussen oder eine vorübergehende Blockierung der enzymatischen Aktivität herbeiführen, wenn es sich bei der angekoppelten Substanz um einen Inhibitor handelt. Dies kann beispielsweise für den Zeitraum der Lagerung sinnvoll sein. Derartige Modifikationen können ferner die Stabilität oder die enzymatische Aktivität beeinflussen. Sie können ferner auch dazu dienen, die Allergenizität und/oder Immunogenizität des Proteins herabzusetzen und damit beispielsweise dessen Hautverträglichkeit zu erhöhen. Beispielsweise können Kopplungen mit makromolekularen Verbindungen, beispielsweise Polyethylenglykol, das Protein hinsichtlich der Stabilität und/oder Hautverträglichkeit verbessern.

Unter Derivaten eines erfindungsgemäßen Proteins können im weitesten Sinne auch Präparationen dieser Proteine verstanden werden. Je nach Gewinnung, Aufarbeitung oder Präparation kann ein Protein mit diversen anderen Stoffen kombiniert sein, beispielsweise aus der Kultur der produzierenden Mikroorganismen. Ein Protein kann auch, beispielsweise zur Erhöhung seiner Lagerstabilität, mit anderen Stoffen gezielt versetzt worden sein. Erfindungsgemäß sind deshalb auch alle Präparationen eines erfindungsgemäßen Proteins. Das ist auch unabhängig davon, ob es in einer bestimmten Präparation tatsächlich diese enzymatische Aktivität entfaltet oder nicht. Denn es kann gewünscht sein, dass es bei der Lagerung keine oder nur geringe Aktivität besitzt, und erst zum Zeitpunkt der Verwendung seine enzymatische Funktion entfaltet. Dies kann beispielsweise über entsprechende Begleitstoffe gesteuert werden. Insbesondere die gemeinsame Präparation von Proteasen mit spezifischen Inhibitoren ist diesbezüglich möglich.

Unter allen vorstehend beschriebenen Proteasen beziehungsweise Proteasevarianten und/oder Derivaten sind im Rahmen der vorliegenden Erfindung diejenigen besonders bevorzugt, deren Lagerstabilität und/oder deren Reinigungsleistung gegenüber der Ausgangsvariante verbessert ist, wobei die Reinigungsleistung in einem Waschsystem bestimmt wird wie vorstehend beschrieben.

Ein weiterer Gegenstand der Erfindung ist eine Nukleinsäure, die für eine erfindungsgemäße Protease codiert, sowie ein Vektor enthaltend eine solche Nukleinsäure, insbesondere ein Klonierungsvektor oder ein Expressionsvektor.

Hierbei kann es sich um DNA- oder RNA-Moleküle handeln. Sie können als Einzelstrang, als ein zu diesem Einzelstrang komplementärer Einzelstrang oder als Doppelstrang vorliegen. Insbesondere bei DNA-Molekülen sind die Sequenzen beider komplementärer Stränge in jeweils allen drei möglichen Leserastern zu berücksichtigen. Ferner ist zu berücksichtigen, dass verschiedene Codons, also Basentriplets, für die gleichen Aminosäuren codieren können, so dass eine bestimmte Aminosäuresequenz von mehreren unterschiedlichen Nukleinsäuren codiert werden kann. Auf Grund dieser Degeneriertheit des genetischen Codes sind sämtliche Nukleinsäuresequenzen in diesem Erfindungsgegenstand eingeschlossen, die eine der vorstehend beschriebenen Proteasen codieren können. Der Fachmann ist in der Lage, diese Nukleinsäuresequenzen zweifelsfrei zu bestimmen, da trotz der Degeneriertheit des genetischen Codes einzelnen Codons definierte Aminosäuren zuzuordnen sind. Daher kann der Fachmann ausgehend von einer Aminosäuresequenz für diese Aminosäuresequenz codierende Nukleinsäuren problemlos ermitteln. Weiterhin können bei erfindungsgemäßen Nukleinsäuren ein oder mehrere Codons durch synonyme Codons ersetzt sein. Dieser Aspekt bezieht sich insbesondere auf die heterologe Expression der erfindungsgemäßen Enzyme. So besitzt jeder Organismus, beispielsweise eine Wirtszelle eines Produktionsstammes, eine bestimmte Codon-Verwendung. Unter Codon-Verwendung wird die Übersetzung des genetischen Codes in Aminosäuren durch den jeweiligen Organismus verstanden. Es kann zu Engpässen in der Proteinbiosynthese kommen, wenn die auf der Nukleinsäure liegenden Codons in dem Organismus einer vergleichsweise geringen Zahl von beladenen tRNA-Molekülen gegenüberstehen. Obwohl für die gleiche Aminosäure codierend führt das dazu, dass in dem Organismus ein Codon weniger effizient translatiert wird als ein synonymes Codon, das für dieselbe Aminosäure codiert. Auf Grund des Vorliegens einer höheren Anzahl von tRNA-Molekülen für das synonyme Codon kann dieses in dem Organismus effizienter translatiert werden.

Einem Fachmann ist es über heutzutage allgemein bekannte Methoden, wie beispielsweise die chemische Synthese oder die Polymerase-Kettenreaktion (PCR) in Verbindung mit molekularbiologischen und/oder proteinchemischen Standardmethoden möglich, anhand bekannter DNA- und/oder Aminosäuresequenzen die entsprechenden Nukleinsäuren bis hin zu vollständigen Genen herzustellen. Derartige Methoden sind beispielsweise aus Sambrook, J., Fritsch, E.F. and Maniatis, T. 2001. Molecular cloning: a laboratory manual, 3. Edition Cold Spring Laboratory Press. bekannt.

Unter Vektoren werden im Sinne der vorliegenden Erfindung aus Nukleinsäuren bestehende Elemente verstanden, die als kennzeichnenden Nukleinsäurebereich eine erfindungsgemäße Nukleinsäure enthalten. Sie vermögen diese in einer Spezies oder einer Zelllinie über mehrere Generationen oder Zellteilungen hinweg als stabiles genetisches Element zu etablieren. Vektoren sind insbesondere bei der Verwendung in Bakterien spezielle Plasmide, also zirkulare genetische Elemente. Im Rahmen der vorliegenden Erfindung wird eine erfindungsgemäße Nukleinsäure in einen Vektor kloniert. Zu den Vektoren zählen beispielsweise solche, deren Ursprung bakterielle Plasmide, Viren oder Bakteriophagen sind, oder überwiegend synthetische Vektoren oder Plasmide mit Elementen verschiedenster Herkunft. Mit den weiteren jeweils vorhandenen genetischen Elementen vermögen Vektoren sich in den betreffenden Wirtszellen über mehrere Generationen hinweg als stabile Einheiten zu etablieren. Sie können extrachromosomal als eigene Einheiten vorliegen oder in ein Chromosom oder chromosomale DNA integrieren.

Expressionsvektoren umfassen Nukleinsäuresequenzen, die sie dazu befähigen, in den sie enthaltenden Wirtszellen, vorzugsweise Mikroorganismen, besonders bevorzugt Bakterien, zu replizieren und dort eine enthaltene Nukleinsäure zur Expression zu bringen. Die Expression wird insbesondere von dem oder den Promotoren beeinflusst, welche die Transkription regulieren. Prinzipiell kann die Expression durch den natürlichen, ursprünglich vor der zu exprimierenden Nukleinsäure lokalisierten Promotor erfolgen, aber auch durch einen auf dem Expressionsvektor bereitgestellten Promotor der Wirtszelle oder auch durch einen modifizierten oder einen völlig anderen Promotor eines anderen Organismus oder einer anderen Wirtszelle. Im vorliegenden Fall wird zumindest ein Promotor für die Expression einer erfindungsgemäßen Nukleinsäure zur Verfügung gestellt und für deren Expression genutzt. Expressionsvektoren können ferner regulierbar sein, beispielsweise durch Änderung der Kultivierungsbedingungen oder bei Erreichen einer bestimmten Zelldichte der sie enthaltenen Wirtszellen oder durch Zugabe von bestimmten Substanzen, insbesondere Aktivatoren der Genexpression. Ein Beispiel für eine solche Substanz ist das Galactose-Derivat Isopropyl-β-D-thiogalactopyranosid (IPTG), welches als Aktivator des bakteriellen Lactose-Operons (lac-Operons) verwendet wird. Im Gegensatz zu Expressionsvektoren wird die enthaltene Nukleinsäure in Klonierungsvektoren nicht exprimiert.

Ein weiterer Gegenstand der Erfindung ist eine nicht-menschliche Wirtszelle, die eine erfindungsgemäße Nukleinsäure oder einen erfindungsgemäßen Vektor beinhaltet, oder die eine erfindungsgemäße Protease beinhaltet, insbesondere eine, die die Protease in das die Wirtszelle umgebende Medium sezerniert. Bevorzugt wird eine erfindungsgemäße Nukleinsäure oder ein erfindungsgemäßer Vektor in einen Mikroorganismus transformiert, der dann eine erfindungsgemäße Wirtszelle darstellt. Alternativ können auch einzelne Komponenten, d.h. Nukleinsäure-Teile oder -Fragmente einer erfindungsgemäßen Nukleinsäure derart in eine Wirtszelle eingebracht werden, dass die dann resultierende Wirtszelle eine erfindungsgemäße Nukleinsäure oder einen erfindungsgemäßen Vektor enthält. Dieses Vorgehen eignet sich besonders dann, wenn die Wirtszelle bereits einen oder mehrere Bestandteile einer erfindungsgemäßen Nukleinsäure oder eines erfindungsgemäßen Vektors enthält und die weiteren Bestandteile dann entsprechend ergänzt werden. Verfahren zur Transformation von Zellen sind im Stand der Technik etabliert und dem Fachmann hinlänglich bekannt. Als Wirtszellen eignen sich prinzipiell alle Zellen, das heißt prokaryotische oder eukaryotische Zellen. Bevorzugt sind solche Wirtszellen, die sich genetisch vorteilhaft handhaben lassen, was beispielsweise die Transformation mit der Nukleinsäure oder dem Vektor und dessen stabile Etablierung angeht, beispielsweise einzellige Pilze oder Bakterien. Ferner zeichnen sich bevorzugte Wirtszellen durch eine gute mikrobiologische und biotechnologische Handhabbarkeit aus. Das betrifft beispielsweise leichte Kultivierbarkeit, hohe Wachstumsraten, geringe Anforderungen an Fermentationsmedien und gute Produktions- und Sekretionsraten für Fremdproteine. Bevorzugte erfindungsgemäße Wirtszellen sezernieren das (transgen) exprimierte Protein in das die Wirtszellen umgebende Medium. Ferner können die Proteasen von den sie produzierenden Zellen nach deren Herstellung modifiziert werden, beispielsweise durch Anknüpfung von Zuckermolekülen, Formylierungen, Aminierungen, usw. Solche posttranslationalen Modifikationen können die Protease funktionell beeinflussen.

Weitere bevorzugte Ausführungsformen stellen solche Wirtszellen dar, die aufgrund genetischer Regulationselemente, die beispielsweise auf dem Vektor zur Verfügung gestellt werden, aber auch von vornherein in diesen Zellen vorhanden sein können, in ihrer Aktivität regulierbar sind. Beispielsweise durch kontrollierte Zugabe von chemischen Verbindungen, die als Aktivatoren dienen, durch Änderung der Kultivierungsbedingungen oder bei Erreichen einer bestimmten Zelldichte können diese zur Expression angeregt werden. Dies ermöglicht eine wirtschaftliche Produktion der erfindungsgemäßen Proteine. Ein Beispiel für eine solche Verbindung ist IPTG wie vorstehend beschrieben.

Bevorzugte Wirtszellen sind prokaryotische oder bakterielle Zellen. Bakterien zeichnen sich durch kurze Generationszeiten und geringe Ansprüche an die Kultivierungsbedingungen aus. Dadurch können kostengünstige Kultivierungsverfahren oder Herstellungsverfahren etabliert werden. Zudem verfügt der Fachmann bei Bakterien in der Fermentationstechnik über einen reichhaltigen Erfahrungsschatz. Für eine spezielle Produktion können aus verschiedensten, im Einzelfall experimentell zu ermittelnden Gründen wie Nährstoffquellen, Produktbildungsrate, Zeitbedarf usw., gramnegative oder grampositive Bakterien geeignet sein.

Bei gramnegativen Bakterien wie beispielsweise *Escherichia coli* wird eine Vielzahl von Proteinen in den periplasmatischen Raum sezerniert, also in das Kompartiment zwischen den beiden die Zellen einschließenden Membranen. Dies kann für spezielle Anwendungen vorteilhaft sein. Ferner können auch gramnegative Bakterien so ausgestaltet werden, dass sie die exprimierten Proteine nicht nur in den periplasmatischen Raum, sondern in das das Bakterium umgebende Medium ausschleusen. Grampositive Bakterien wie beispielsweise Bacilli oder Actinomyceten oder andere Vertreter der *Actinomycetales* besitzen demgegenüber keine äußere Membran, so dass sezernierte Proteine sogleich in das die Bakterien umgebende Medium, in der Regel das Nährmedium, abgegeben werden, aus welchem sich die exprimierten Proteine aufreinigen lassen. Sie können aus dem Medium direkt isoliert oder weiter prozessiert werden. Zudem sind grampositive Bakterien mit den meisten Herkunftsorganismen für technisch wichtige Enzyme verwandt oder identisch und bilden meist selbst vergleichbare Enzyme, so dass sie über eine ähnliche Codon-Verwendung verfügen und ihr Protein-Syntheseapparat naturgemäß entsprechend ausgerichtet ist.

Erfindungsgemäße Wirtszellen können hinsichtlich ihrer Anforderungen an die Kulturbedingungen verändert sein, andere oder zusätzliche Selektionsmarker aufweisen oder noch andere oder zusätzliche Proteine exprimieren. Es kann sich insbesondere auch um solche Wirtszellen handeln, die mehrere Proteine oder Enzyme transgen exprimieren.

Die vorliegende Erfindung ist prinzipiell auf alle Mikroorganismen, insbesondere auf alle fermentierbaren Mikroorganismen, besonders bevorzugt auf solche der Gattung *Bacillus,* anwendbar und führt dazu, dass sich durch den Einsatz solcher Mikroorganismen erfindungsgemäße Proteine herstellen lassen. Solche Mikroorganismen stellen dann Wirtszellen im Sinne der Erfindung dar.

In einer weiteren Ausführungsform der Erfindung ist die Wirtszelle dadurch gekennzeichnet, dass sie ein Bakterium ist, bevorzugt eines, das ausgewählt ist aus der Gruppe der Gattungen von *Escherichia, Klebsiella, Bacillus, Staphylococcus, Corynebacterium, Arthrobacter, Streptomyces, Stenotrophomonas* und *Pseudomonas,* weiter bevorzugt eines, das ausgewählt ist aus der Gruppe von *Escherichia coli, Klebsiella planticola, Bacillus licheniformis, Bacillus lentus, Bacillus amyloliquefaciens, Bacillus subtilis, Bacillus alcalophilus, Bacillus globigii, Bacillus gibsonii, Bacillus clausii, Bacillus halodurans, Bacillus pumilus, Staphylococcus carnosus, Corynebacterium glutamicum, Arthrobacter oxidans, Streptomyces lividans, Streptomyces coelicolor* und *Stenotrophomonas maltophilia.*

Die Wirtszelle kann aber auch eine eukaryotische Zelle sein, die dadurch gekennzeichnet ist, dass sie einen Zellkern besitzt. Einen weiteren Gegenstand der Erfindung stellt daher eine Wirtszelle dar, die dadurch gekennzeichnet ist, dass sie einen Zellkern besitzt. Im Gegensatz zu prokaryotischen Zellen sind eukaryotische Zellen in der Lage, das gebildete Protein posttranslational zu modifizieren. Beispiele dafür sind Pilze wie Actinomyceten oder Hefen wie *Saccharomyces* oder *Kluyveromyces.* Dies kann beispielsweise dann besonders vorteilhaft sein, wenn die Proteine im Zusammenhang mit ihrer Synthese spezifische Modifikationen erfahren sollen, die derartige Systeme ermöglichen. Zu den Modifikationen, die eukaryotische Systeme besonders im Zusammenhang mit der Proteinsynthese durchführen, gehören beispielsweise die Bindung niedermolekularer Verbindungen wie Membrananker oder Oligosaccharide. Derartige Oligosaccharid-Modifikationen können beispielsweise zur Senkung der Allergenizität eines exprimierten Proteins wünschenswert sein. Auch eine Co-Expression mit den natürlicherweise von derartigen Zellen gebildeten Enzymen, wie beispielsweise Cellulasen, kann vorteilhaft sein. Ferner können sich beispielsweise thermophile pilzliche Expressionssysteme besonders zur Expression temperaturbeständiger Proteine oder Varianten eignen.

Die erfindungsgemäßen Wirtszellen werden in üblicher Weise kultiviert und fermentiert, beispielsweise in diskontinuierlichen oder kontinuierlichen Systemen. Im ersten Fall wird ein geeignetes Nährmedium mit den Wirtszellen beimpft und das Produkt nach einem experimentell zu ermittelnden Zeitraum aus dem Medium geerntet. Kontinuierliche Fermentationen zeichnen sich durch Erreichen eines Fließgleichgewichts aus, in dem über einen vergleichsweise langen Zeitraum Zellen teilweise absterben aber auch nachwachsen und gleichzeitig aus dem Medium das gebildete Protein entnommen werden kann.

Erfindungsgemäße Wirtszellen werden bevorzugt verwendet, um erfindungsgemäße Proteasen herzustellen. Ein weiterer Gegenstand der Erfindung ist daher ein Verfahren zur Herstellung einer Protease umfassend
a) Kultivieren einer erfindungsgemäßen Wirtszelle, und
b) Isolieren der Protease aus dem Kulturmedium oder aus der Wirtszelle.

Dieser Erfindungsgegenstand umfasst bevorzugt Fermentationsverfahren. Fermentationsverfahren sind an sich aus dem Stand der Technik bekannt und stellen den eigentlichen großtechnischen Produktionsschritt dar, in der Regel gefolgt von einer geeigneten Aufreinigungsmethode des hergestellten Produktes, beispielsweise der erfindungsgemäßen Proteasen. Alle Fermentationsverfahren, die auf einem entsprechenden Verfahren zur Herstellung einer erfindungsgemäßen Protease beruhen, stellen Ausführungsformen dieses Erfindungsgegenstandes dar.

Fermentationsverfahren, die dadurch gekennzeichnet sind, dass die Fermentation über eine Zulaufstrategie durchgeführt wird, kommen insbesondere in Betracht. Hierbei werden die Medienbestandteile, die durch die fortlaufende Kultivierung verbraucht werden, zugefüttert. Hierdurch können beträchtliche Steigerungen sowohl in der Zelldichte als auch in der Zellmasse beziehungsweise Trockenmasse und/oder insbesondere in der Aktivität der interessierenden Protease erreicht werden. Ferner kann die Fermentation auch so gestaltet werden, dass unerwünschte Stoffwechselprodukte herausgefiltert oder durch Zugabe von Puffer oder jeweils passende Gegenionen neutralisiert werden.

Die hergestellte Protease kann aus dem Fermentationsmedium geerntet werden. Ein solches Fermentationsverfahren ist gegenüber einer Isolation der Protease aus der Wirtszelle, d.h. einer Produktaufbereitung aus der Zellmasse (Trockenmasse) bevorzugt, erfordert jedoch die Zurverfügungstellung von geeigneten Wirtszellen oder von einem oder mehreren geeigneten Sekretionsmarkern oder -mechanismen und/oder Transportsystemen, damit die Wirtszellen die Protease in das Fermentationsmedium sezernieren. Ohne Sekretion kann alternativ die Isolation der Protease aus der Wirtszelle, d.h. eine Aufreinigung derselben aus der Zellmasse, erfolgen, beispielsweise durch Fällung mit Ammoniumsulfat oder Ethanol, oder durch chromatographische Reinigung.

Alle vorstehend ausgeführten Sachverhalte können zu Verfahren kombiniert werden, um erfindungsgemäße Protease herzustellen.

Ein weiterer Gegenstand der Erfindung ist ein Mittel, das dadurch gekennzeichnet ist, dass es eine erfindungsgemäße Protease wie vorstehend beschrieben enthält. Bevorzugt ist das Mittel ein Wasch- oder Reinigungsmittel.

Zu diesem Erfindungsgegenstand zählen alle denkbaren Wasch- oder Reinigungsmittelarten, sowohl Konzentrate als auch unverdünnt anzuwendende Mittel, zum Einsatz im kommerziellen Maßstab, in der Waschmaschine oder bei der Handwäsche bzw. -reinigung. Dazu gehören beispielsweise Waschmittel für Textilien, Teppiche, oder Naturfasern, für die die Bezeichnung Waschmittel verwendet wird. Dazu gehören beispielsweise auch Geschirrspülmittel für Geschirrspülmaschinen oder manuelle Geschirrspülmittel oder Reiniger für harte Oberflächen wie Metall, Glas, Porzellan, Keramik, Kacheln, Stein, lackierte Oberflächen, Kunststoffe, Holz oder Leder, für die die Bezeichnung Reinigungsmittel verwendet wird, also neben manuellen und maschinellen Geschirrspülmitteln beispielsweise auch Scheuermittel, Glasreiniger, WC-Duftspüler, usw. Zu den Wasch- und Reinigungsmitteln im Rahmen der Erfindung zählen ferner Waschhilfsmittel, die bei der manuellen oder maschinellen Textilwäsche zum eigentlichen Waschmittel hinzudosiert werden, um eine weitere Wirkung zu erzielen. Ferner zählen zu Wasch- und Reinigungsmittel im Rahmen der Erfindung auch Textilvor- und -nachbehandlungsmittel, also solche Mittel, mit denen das Wäschestück vor der eigentlichen Wäsche in Kontakt gebracht wird, beispielsweise zum Anlösen hartnäckiger Verschmutzungen, und auch solche Mittel, die in einem der eigentlichen Textilwäsche nachgeschalteten Schritt dem Waschgut weitere wünschenswerte Eigenschaften wie angenehmen Griff, Knitterfreiheit oder geringe statische Aufladung verleihen. Zu letztgenannten Mittel werden u.a. die Weichspüler gerechnet.

Die erfindungsgemäßen Wasch- oder Reinigungsmittel, die als pulverförmige Feststoffe, in nachverdichteter Teilchenform, als Gele, homogene Lösungen oder Suspensionen vorliegen können, können neben einer erfindungsgemäßen Protease alle bekannten und in derartigen Mitteln üblichen Inhaltsstoffe enthalten, wobei bevorzugt mindestens ein weiterer Inhaltsstoff in dem Mittel vorhanden ist. Die erfindungsgemäßen Mittel können insbesondere Tenside, Builder (Gerüststoffe), Persauerstoffverbindungen oder Bleichaktivatoren enthalten. Ferner können sie wassermischbare organische Lösungsmittel, weitere Enzyme, Sequestrierungsmittel, Elektrolyte, pH-Regulatoren und/oder weitere Hilfsstoffe wie optische Aufheller, Vergrauungsinhibitoren, Schaumregulatoren sowie Farb- und Duftstoffe sowie Kombinationen hiervon enthalten.

Insbesondere eine Kombination einer erfindungsgemäßen Protease mit einem oder mehreren weiteren Inhaltsstoff(en) des Mittels ist vorteilhaft, da ein solches Mittel in bevorzugten erfindungsgemäßen Ausgestaltungen eine verbesserte Reinigungsleistung durch sich ergebende Synergismen aufweist. Insbesondere durch die Kombination einer erfindungsgemäßen Protease mit einem Tensid und/oder einem Builder (Gerüststoff) und/oder einer Persauerstoffverbindung und/oder einem Bleichaktivator kann ein solcher Synergismus erreicht werden. Allerdings kann in bevorzugten Ausführungsformen das erfindungsgemäße Mittel keine Borsäure enthalten.

Vorteilhafte Inhaltsstoffe erfindungsgemäßer Mittel sind offenbart in der internationalen Patentanmeldung WO2009/121725A1, dort beginnend auf Seite 5, vorletzter Absatz, und endend auf Seite 13 nach dem zweiten Absatz.

Ein erfindungsgemäßes Mittel enthält die Protease vorteilhafterweise in einer Menge von 2µg bis 20mg, vorzugsweise von 5µg bis 17,5mg, besonders bevorzugt von 20µg bis 15mg und ganz besonders bevorzugt von 50µg bis 10mg pro g des Mittels. In verschiedenen Ausführungsformen beträgt die Konzentration der hierin beschriebenen Protease (aktives Enzym) in dem Mittel >0 bis 1 Gew.-%, vorzugsweise 0,001 bis 0,1 Gew.-% bezogen auf das Gesamtgewicht des Mittels oder der Zusammensetzung. Ferner kann die in dem Mittel enthaltene Protease und/oder weitere Inhaltsstoffe des Mittels mit einer bei Raumtemperatur oder bei Abwesenheit von Wasser für das Enzym undurchlässigen Substanz umhüllt sein, welche unter Anwendungsbedingungen des Mittels durchlässig für das Enzym wird. Eine solche Ausführungsform der Erfindung ist somit dadurch gekennzeichnet, dass die Protease mit einer bei Raumtemperatur oder bei Abwesenheit von Wasser für die Protease undurchlässigen Substanz umhüllt ist. Weiterhin kann auch das Wasch- oder Reinigungsmittel selbst in einem Behältnis, vorzugsweise einem luftdurchlässigen Behältnis, verpackt sein, aus dem es kurz vor Gebrauch oder während des Waschvorgangs freigesetzt wird.

In weiteren Ausführungsformen der Erfindung ist das Mittel dadurch gekennzeichnet, dass es
(a) in fester Form vorliegt, insbesondere als rieselfähiges Pulver mit einem Schüttgewicht von 300 g/l bis 1200 g/l, insbesondere 500 g/l bis 900 g/l, oder
(b) in pastöser oder in flüssiger Form vorliegt, und/oder
(c) in gelförmiger oder dosierbeutelförmiger (Pouches) Form vorliegt, und/oder
(d) als Einkomponentensystem vorliegt, oder
(e) in mehrere Komponenten aufgeteilt ist.

Diese Ausführungsformen der vorliegenden Erfindung umfassen alle festen, pulverförmigen, flüssigen, gelförmigen oder pastösen Darreichungsformen erfindungsgemäßer Mittel, die gegebenenfalls auch aus mehreren Phasen bestehen können sowie in komprimierter oder nicht komprimierter Form vorliegen können. Das Mittel kann als rieselfähiges Pulver vorliegen, insbesondere mit einem Schüttgewicht von 300 g/l bis 1200 g/l, insbesondere 500 g/l bis 900 g/l oder 600 g/l bis 850 g/l. Zu den festen Darreichungsformen des Mittels zählen ferner Extrudate, Granulate, Tabletten oder Pouches. Alternativ kann das Mittel auch flüssig, gelförmig oder pastös sein, beispielsweise in Form eines nicht-wässrigen Flüssigwaschmittels oder einer nicht-wässrigen Paste oder in Form eines wässrigen Flüssigwaschmittels oder einer wasserhaltigen Paste. Flüssige Mittel sind generell bevorzugt. Weiterhin kann das Mittel als Einkomponentensystem vorliegen. Solche Mittel bestehen aus einer Phase. Alternativ kann ein Mittel auch aus mehreren Phasen bestehen. Ein solches Mittel ist demnach in mehrere Komponenten aufgeteilt.

Erfindungsgemäße Wasch- oder Reinigungsmittel können ausschließlich eine Protease enthalten. Alternativ können sie auch weitere hydrolytische Enzyme oder andere Enzyme in einer für die Wirksamkeit des Mittels zweckmäßigen Konzentration enthalten. Eine weitere Ausführungsform der Erfindung stellen somit Mittel dar, die ferner eines oder mehrere weitere Enzyme umfassen. Als weitere Enzyme bevorzugt einsetzbar sind alle Enzyme, die in dem erfindungsgemäßen Mittel eine katalytische Aktivität entfalten können, insbesondere eine Lipase, Amylase, Cellulase, Hemicellulase, Mannanase, Tannase, Xylanase, Xanthanase, Xyloglucanase, β-Glucosidase, Pektinase, Carrageenase, Perhydrolase, Oxidase, Oxidoreduktase oder eine andere - von den erfindungsgemäßen Proteasen unterscheidbare - Protease sowie deren Gemische. Weitere Enzyme sind in dem Mittel vorteilhafterweise jeweils in einer Menge von 1×10⁻⁸ bis 5 Gew.-% bezogen auf aktives Protein enthalten. Zunehmend bevorzugt ist jedes weitere Enzym in einer Menge von 1×10⁻⁷ bis 3 Gew.-%, von 0,00001 bis 1 Gew.-%, von 0,00005 bis 0,5 Gew.-%, von 0,0001 bis 0,1 Gew.-% und besonders bevorzugt von 0,0001 bis 0,05 Gew.-% in erfindungsgemäßen Mitteln enthalten, jeweils bezogen auf aktives Protein. Besonders bevorzugt zeigen die Enzyme synergistische Reinigungsleistungen gegenüber bestimmten Anschmutzungen oder Flecken, d.h. die in der Mittelzusammensetzung enthaltenen Enzyme unterstützen sich in ihrer Reinigungsleistung gegenseitig. Ganz besonders bevorzugt liegt ein solcher Synergismus vor zwischen der erfindungsgemäß enthaltenen Protease und einem weiteren Enzym eines erfindungsgemäßen Mittels, darunter insbesondere zwischen der genannten Protease und einer Amylase und/oder einer Lipase und/oder einer Mannanase und/oder einer Cellulase und/oder einer Pektinase. Synergistische Effekte können nicht nur zwischen verschiedenen Enzymen, sondern auch zwischen einem oder mehreren Enzymen und weiteren Inhaltsstoffen des erfindungsgemäßen Mittels auftreten.

In den hierin beschriebenen Reinigungsmitteln können die einzusetzenden Enzyme ferner zusammen mit Begleitstoffen, etwa aus der Fermentation, konfektioniert sein. In flüssigen Formulierungen werden die Enzyme bevorzugt als Enzymflüssigformulierung(en) eingesetzt.

Die Enzyme werden in der Regel nicht in Form des reinen Proteins, sondern vielmehr in Form stabilisierter, lager- und transportfähiger Zubereitungen bereitgestellt. Zu diesen vorkonfektionierten Zubereitungen zählen beispielsweise die durch Granulation, Extrusion oder Lyophilisierung erhaltenen festen Präparationen oder, insbesondere bei flüssigen oder gelförmigen Mitteln, Lösungen der Enzyme, vorteilhafterweise möglichst konzentriert, wasserarm und/oder mit Stabilisatoren oder weiteren Hilfsmitteln versetzt.

Alternativ können die Enzyme sowohl für die feste als auch für die flüssige Darreichungsform verkapselt werden, beispielsweise durch Sprühtrocknung oder Extrusion der Enzymlösung zusammen mit einem vorzugsweise natürlichen Polymer oder in Form von Kapseln, beispielsweise solchen, bei denen die Enzyme wie in einem erstarrten Gel eingeschlossen sind oder in solchen vom Kern-Schale-Typ, bei dem ein enzymhaltiger Kern mit einer Wasser-, Luft- und/oder Chemikalienundurchlässigen Schutzschicht überzogen ist. In aufgelagerten Schichten können zusätzlich weitere Wirkstoffe, beispielsweise Stabilisatoren, Emulgatoren, Pigmente, Bleich- oder Farbstoffe aufgebracht werden. Derartige Kapseln werden nach an sich bekannten Methoden, beispielsweise durch Schüttel- oder Rollgranulation oder in Fluid-bed-Prozessen aufgebracht. Vorteilhafterweise sind derartige Granulate, beispielsweise durch Aufbringen polymerer Filmbildner, staubarm und aufgrund der Beschichtung lagerstabil.

Weiterhin ist es möglich, zwei oder mehrere Enzyme zusammen zu konfektionieren, so dass ein einzelnes Granulat mehrere Enzymaktivitäten aufweist.

Die Enzyme können auch in wasserlösliche Filme, wie sie beispielsweise bei der Konfektionierung von Wasch- und Reinigungsmitteln in Einheitsdosisform verwendet werden, eingebracht werden. Ein derartiger Film ermöglicht die Freisetzung der Enzyme nach Kontakt mit Wasser. Wie hierin verwendet, bezieht sich "wasserlöslich" auf eine Filmstruktur, die vorzugsweise vollständig wasserlöslich ist. Bevorzugt besteht ein solcher Film aus (vollständig oder teilweise hydrolysiertem) Polyvinylalkohol (PVA).

Ein weiterer Erfindungsgegenstand ist ein Verfahren zur Reinigung von Textilien oder harten Oberflächen, das dadurch gekennzeichnet ist, dass in mindestens einem Verfahrensschritt ein erfindungsgemäßes Mittel angewendet wird, oder dass in mindestens einem Verfahrensschritt eine erfindungsgemäße Protease katalytisch aktiv wird, insbesondere derart, dass die Protease in einer Menge von 40µg bis 4g, vorzugsweise von 50µg bis 3g, besonders bevorzugt von 100µg bis 2g und ganz besonders bevorzugt von 200µg bis 1g bzw. in den hierin beschriebenen Konzentrationen eingesetzt wird.

In verschiedenen Ausführungsformen zeichnet sich das oben beschriebene Verfahren dadurch aus, dass die Protease bei einer Temperatur von 0 bis 100°C, bevorzugt von 0 bis 60°C, weiter bevorzugt von 20 bis 40°C und am meisten bevorzugt bei 25°C eingesetzt wird.

Hierunter fallen sowohl manuelle als auch maschinelle Verfahren, wobei maschinelle Verfahren bevorzugt sind. Verfahren zur Reinigung von Textilien zeichnen sich im Allgemeinen dadurch aus, dass in mehreren Verfahrensschritten verschiedene reinigungsaktive Substanzen auf das Reinigungsgut aufgebracht und nach der Einwirkzeit abgewaschen werden, oder dass das Reinigungsgut in sonstiger Weise mit einem Waschmittel oder einer Lösung oder Verdünnung dieses Mittels behandelt wird. Entsprechendes gilt für Verfahren zur Reinigung von allen anderen Materialien als Textilien, insbesondere von harten Oberflächen. Alle denkbaren Wasch- oder Reinigungsverfahren können in wenigstens einem der Verfahrensschritte um die Anwendung eines erfindungsgemäßen Wasch- oder Reinigungsmittels oder einer erfindungsgemäßen Protease bereichert werden und stellen dann Ausführungsformen der vorliegenden Erfindung dar. Alle Sachverhalte, Gegenstände und Ausführungsformen, die für erfindungsgemäße Protease und sie enthaltende Mittel beschrieben sind, sind auch auf diesen Erfindungsgegenstand anwendbar. Daher wird an dieser Stelle ausdrücklich auf die Offenbarung an entsprechender Stelle verwiesen mit dem Hinweis, dass diese Offenbarung auch für die vorstehenden erfindungsgemäßen Verfahren gilt.

Da erfindungsgemäße Proteasen natürlicherweise bereits eine hydrolytische Aktivität besitzen und diese auch in Medien entfalten, die sonst keine Reinigungskraft besitzen wie beispielsweise in bloßem Puffer, kann ein einzelner und/oder der einzige Schritt eines solchen Verfahrens darin bestehen, dass als einzige reinigungsaktive Komponente eine erfindungsgemäße Protease mit der Anschmutzung in Kontakt gebracht wird, bevorzugt in einer Pufferlösung oder in Wasser. Dies stellt eine weitere Ausführungsform dieses Erfindungsgegenstandes dar.

Alternative Ausführungsformen dieses Erfindungsgegenstandes stellen auch Verfahren zur Behandlung von Textilrohstoffen oder zur Textilpflege dar, bei denen in wenigstens einem Verfahrensschritt eine erfindungsgemäße Protease aktiv wird. Hierunter sind Verfahren für Textilrohstoffe, Fasern oder Textilien mit natürlichen Bestandteilen bevorzugt, und ganz besonders für solche mit Wolle oder Seide.

Schließlich erfasst die Erfindung auch die Verwendung der hierin beschriebenen Proteasen in Wasch- oder Reinigungsmitteln, beispielsweise wie oben beschrieben, zur (verbesserten) Entfernung von proteinhaltigen Anschmutzungen, beispielsweise von Textilien oder harten Oberflächen. In bevorzugten Ausführungsformen dieser Verwendung wird die Protease im Wasch- oder Reinigungsmittel vor einem Wasch- oder Reinigungsvorgang 3 oder mehr Tage, 4 oder mehr Tage, 7 oder mehr Tage, 10 oder mehr Tage, 12 oder mehr Tage, 14 oder mehr Tage, 21 oder mehr Tage oder 28 oder mehr Tage gelagert.

Alle Sachverhalte, Gegenstände und Ausführungsformen, die für erfindungsgemäße Protease und sie enthaltende Mittel beschrieben sind, sind auch auf diesen Erfindungsgegenstand anwendbar. Daher wird an dieser Stelle ausdrücklich auf die Offenbarung an entsprechender Stelle verwiesen mit dem Hinweis, dass diese Offenbarung auch für die vorstehende erfindungsgemäße Verwendung gilt.

### Beispiele

### Beispiel1: Übersicht über die Mutationen

Die Erfindung betrifft eine alkalische Protease vom Subtilisin Typ aus *Bacillus lentus.* Von einer Ausgangsvariante (Protease gemäß SEQ ID NO:2 aus WO2013/060621A1) wurden durch Zufallsmutagenese Varianten hergestellt, welche dann u.a. auf verbesserte Waschleistung und/oder Enzymstabilität gescreent wurden. Auf diesem Weg wurde aus der genannten Protease 27 Mutanten mit verbesserter Lagerstabilität und/oder verbesserter Reinigungsleistung generiert.

| **Variante** | **Aminosäuresubstitutionen relativ zu SEQ ID NO:1** | | | | | | |
|---|---|---|---|---|---|---|---|
| Ausgangsvariant e | S3T | V4I | R99E | V199I | | | |
| Mutante 1 | S3T | V4I | R99E | V199I | A267S | N255Y | |
| Mutante 2 | S3T | V4I | R99E | V199I | N198D | S234A | L21F |
| Mutante 3 | S3T | V4I | R99E | V199I | N212D | | |
| Mutante 4 | S3T | V4I | R99E | V199I | G205D | | |
| Mutante 5 | S3T | V4I | R99E | V199I | S182C | | |
| Mutante 6 | S3T | V4I | R99E | V199I | R44S | S234A | |
| Mutante 7 | S3T | V4I | R99E | V199I | S253C | | |
| Mutante 8 | S3T | V4I | R99E | V199I | I105V | | |
| Mutante 9 | S3T | V4I | R99E | V199I | N177D | | |
| Mutante 10 | S3T | V4I | R99E | V199I | V193M | | |
| Mutante 11 | S3T | V4I | R99E | V199I | S9C | | |
| Mutante 12 | S3T | V4I | R99E | V199I | N198D | | |
| Mutante 13 | S3T | V4I | R99E | V199I | S259C | | |
| Mutante 14 | S3T | V4I | R99E | V199I | I159L | Q230E | |
| Mutante 15 | S3T | V4I | R99E | V199I | P204L | | |
| Mutante 16 | S3T | V4I | R99E | V199I | L21F | | |
| Mutante 17 | S3T | V4I | R99E | V199I | N42H | | |
| Mutante 18 | S3T | V4I | R99E | V199I | S139R | S250N | |
| Mutante 19 | S3T | V4I | R99E | V199I | N42S | | |
| Mutante 20 | S3T | V4I | R99E | V199I | V137I | | |
| Mutante 21 | S3T | V4I | R99E | V199I | V145I | | |
| Mutante 22 | S3T | V4I | R99E | V199I | S206A | | |
| Mutante 23 | S3T | V4I | R99E | V199I | G113A | L21W | |
| Mutante 24 | S3T | V4I | R99E | V199I | A112V | | |
| Mutante 25 | S3T | V4I | R99E | V199I | A168V | | |
| Mutante 26 | S3T | V4I | R99E | V199I | Q176E | A131D | |
| Mutante 27 | S3T | V4I | R99E | V199I | T141S | S210C | |

### Verwendete Waschmittelmatrix

| Chemischer Name | Gew.-% Aktivsubstanz in der Formulierung |
|---|---|
| Wasser demin. | Rest |
| Na-LAS | 3-8 |
| Anionische Tenside | 4-6 |
| Sodiumlaurethsulfat | 2,0 |
| Nicht-ionische Tenside | 2-6 |
| Phosphonat | 0,2-0,6 |
| Zitronensäure | 0,2-2 |
| NaOH | 1-4 |
| Entschäumer | < 1 |
| Glycerin | 0,5-2 |
| Konservierungsmittel | < 0,1 |
| Ohne opt. Aufheller, Parfüm, Farbstoff und Enzyme. | |
| Dosierung: 6 g/L | |

### Verwendete Geschirrspülmittelmatrix

| Chemischer Name | Gew.-% Aktivsubstanz in der Formulierung |
|---|---|
| Alkylbenzolsulfonsäure | 8-20 |
| Wasser | 30-80 |
| NaOH (50%) | 5,4 |
| Fettalkoholethersulfat | 7,14 |
| NaCl-Lsg (20%) | 2,0 |
| Phosphorsäure (H₃PO₄) (34%/85%) | 0,383 (34%) |
| Konservierungsmittel | 0,1 |
| Parfüm | 0,25 |
| Farbstoff | 1,0 |
| Bitterstoff | 0,04 |

### Beispiel 2: Bestimmung der Lagerstabilität

### Lagerung

Die erfindungsgemäßen Proteasevarianten wurden in *Bacillus subtilis* exprimiert. Aus den Überständen der *Bacillus subtilis* Kultur wurden die Proteasen auf ein gleiches Aktivitätslevel verdünnt. Zum Bestimmen der Lagerstabilität in Waschmittel bzw. Geschirrspülmittel wurden 50% Waschmittelmatrix ohne Borsäure bzw. 50% Spülmittelmatrix mit 50% entsprechend verdünnter *Bacillus subtilis* Kultur versetzt und gut gemischt. Die verschlossenen Gefäße wurden jeweils für drei bzw. 3,5 Wochen bei 40°C gelagert. Die entnommene Probenmenge wurde für 20 min bei Raumtemperatur in 0,1M Tris/HCl (pH 8,6) durch Rühren gelöst. Danach wurde der AAPF Assay wie unten beschrieben durchgeführt.

### Protease-Aktivitäts-Assay

Die Aktivität der Protease wird durch die Freisetzung des Chromophors para-Nitroanilin aus dem Substrat Succinyl Alanin-Alanin-Prolin-Phenylalanin-para-Nitroanilid (AAPFpNA; Bachem L-1400) bestimmt. Die Freisetzung des pNA verursacht eine Zunahme der Extinktion bei 410nm, deren zeitlicher Verlauf ein Maß für die enzymatische Aktivität ist.

Die Messung erfolgte bei einer Temperatur von 25°C, pH 8,6 und einer Wellenlänge von 410nm. Die Messzeit betrug 5 min bei einem Messintervall von 20 bis 60 Sekunden.

### Messansatz:

10 µL AAPF-Lösung (70 mg/mL)
1000 µL Tris/HCl (0,1 M; pH 8,6 mit 0,1 % Brij 35)
10 µL verdünnte Protease-Lösung
Kinetik erstellt über 5 min bei 25°C (410nm)

Im Folgenden ist die verbesserte Stabilität in % gegenüber der Stabilität der Ausgangsvariante nach 3 Wochen Lagerung bei 40°C in der o.g. Waschmittel- bzw. Geschirrspülmittelmatrix gezeigt:

| Variante | Verbesserte Stabilität (%) in o.g. Waschmittelmatrix | Verbesserte Stabilität (%) in o.g. Geschirrspülmittelmatrix |
|---|---|---|
| Ausgangsvariante | 0 | 0 |
| Mutante 1 | +44 | +14 |
| Mutante 2 | +49 | +55 |
| Mutante 3 | +93 | +41 |
| Mutante 4 | +57 | +5 |
| Mutante 5 | +80 | +11 |
| Mutante 6 | +58 | +15 |
| Mutante 7 | +9 | +41 |
| Mutante 8 | +102 | +58 |
| Mutante 9 | +65 | +52 |
| Mutante 10 | +43 | +35 |
| Mutante 11 | +67 | +25 |
| Mutante 12 | +60 | +19 |
| Mutante 13 | +54 | +28 |
| Mutante 14 | +64 | +9 |
| Mutante 15 | +56 | +16 |
| Mutante 16 | +59 | +0 |
| Mutante 17 | +64 | +22 |
| Mutante 18 | +42 | +2 |
| Mutante 19 | +46 | +29 |
| Mutante 20 | +58 | +4 |
| Mutante 21 | +73 | +12 |
| Mutante 22 | +63 | +33 |
| Mutante 23 | +71 | +22 |
| Mutante 24 | +47 | +5 |
| Mutante 25 | +38 | +24 |
| Mutante 26 | +68 | +62 |
| Mutante 27 | +77 | +48 |

Alle Varianten zeigen in Waschmittel und/oder Geschirrspülmittel eine verbesserte Stabilität im Vergleich zur Ausgangsvariante.

### Beispiel 3: Bestimmung der Reinigungsleistung

### Miniwaschtest

Waschtest mit *Bacillus subtilis* Kulturüberständen, welche die gescreenten Protease-Mutanten durch heterologe Expression enthalten. Die Überstände werden aktivitätsgleich zum Benchmark = Ausgangsvariante mit einer marktüblichen Konzentration für Proteasen in Waschmittel eingesetzt. Anders als bei der Bestimmung der Lagerstabilität werden die Proben nicht gelagert, sondern die Reinigungsleistung wird direkt bestimmt. Die Mutanten werden alle auf die Waschleistung der Ausgangsvariante bezogen, die gleich 100% gesetzt wird (Summe der 7 Anschmutzungen, korrigiert durch die Leistung des Waschmittels allein).

### Bedingungen: 40°C, 16° dH Wasser, 1h

### Anschmutzungen:

1. CFT CS038
2. CFT PC-10
3. WfK 10N
4. CFT C-03
5. EMPA 112
6. CFT C-05
7. H-MR-B

Ausgestanzte Gewebe (Durchmesser = 10 mm) wurden in Mikrotiterplatten vorgelegt, Waschlauge auf 40°C vortemperiert, Endkonzentration 3,17 g/L, Lauge und Enzym auf die Anschmutzung gegeben, für 1h bei 40°C und 600 rpm inkubiert, anschließend die Anschmutzung mehrmals mit klarem Wasser gespült, trocknen gelassen und mit einem Farbmessgerät die Helligkeit bestimmt. Je heller das Gewebe wird, desto besser ist die Reinigungsleistung. Gemessen wird hier der L-Wert = Helligkeit, je höher desto heller. Angegeben ist die Summe der 7 Anschmutzungen in % bezogen auf die Ausgangsvariante korrigiert durch die Leistung des Waschmittels ohne Protease.

| **Variante** | **Leistung im Waschtest bei 40°C** (bezogen auf Leistung der Ausgangsvariante) |
|---|---|
| Ausgangsvariante | 100% |
| Mutante 10 | 104% |
| Mutante 13 | 104% |

Die Varianten 10 und 13 zeigen eine erhöhte Waschleistung im Vergleich zur Ausgangsvariante.

Erfindungsgemäße Proteasen, insbesondere die Varianten 10 und 13, zeigen folglich nicht nur eine verbesserte Lagerstabilität, sondern auch eine verbesserte Reinigungsleistung.

## Patentansprüche

1. Protease, umfassend eine Aminosäuresequenz, die mindestens 70% Sequenzidentität mit der in SEQ ID NO:1 angegebenen Aminosäuresequenz über deren Gesamtlänge aufweist und die bezogen auf die Nummerierung gemäß SEQ ID NO:1 an
i) den Positionen, die den Positionen 3, 4, 99 und 199 entsprechen, die Aminosäuresubstitutionen S3T, V4I, R99E und V199I aufweist, und
ii) mindestens einer der Positionen, die den Positionen 9, 21, 42, 44, 105, 112, 113, 131, 137, 139, 141, 145, 159, 168, 176, 177, 182, 193, 198, 204, 205, 206, 210, 212, 230, 234, 250, 253, 255, 259 oder 267 entsprechen, mindestens eine Aminosäuresubstitution aufweist.

2. Protease nach Anspruch 1, wobei
die mindestens eine Aminosäuresubstitution an mindestens einer der Positionen, die den Positionen 9, 21, 42, 44, 105, 112, 113, 131, 137, 139, 141, 145, 159, 168, 176, 177, 182, 193, 198, 204, 205, 206, 210, 212, 230, 234, 250, 253, 255, 259 oder 267 entsprechen, aus der aus 9C, 21F, 21W, 42S, 42H, 44S, 105V, 112V, 113A, 131D, 137I, 139R, 141S, 145I, 159L, 168V, 176E, 177D, 182C, 193M, 198D, 204L, 205D, 206A, 210C, 212D, 230E, 234A, 250N, 253C, 255Y, 259C und 267S bestehenden Gruppe ausgewählt ist.

3. Protease nach einem der Ansprüche 1 bis 2, wobei die Protease, jeweils bezogen auf die Nummerierung gemäß SEQ ID NO:1, aufweist:
i) die Aminosäuresubstitutionen S3T + V4I + R99E + V199I und
ii) eine der folgenden Aminosäuresubstitutionskombinationen: (i) A267S + N255Y; (ii) N198D + S234A + L21F; (iii) N212D; (iv) G205D; (v) S182C; (vi) R44S + S234A; (vii) S253C; (viii) I105V;(ix) N177D; (x) V193M; (xi) S9C; (xii) N198D; (xiii) S259C; (xiv) I159L + Q230E; (xv) P204L; (xvi) L21F; (xvii) N42H; (xviii) S139R + S250N; (xix) N42S; (xx) V137I; (xxi) V145I; (xxii) S206A; (xxiii) G113A + L21W; (xxiv) A112V; (xxv) A168V; (xxvi) Q176E + A131D; (xxvii) T141S + S210C.

4. Protease, **dadurch gekennzeichnet, dass**
(a) sie aus einer Protease nach einem der Ansprüche 1 bis 3 als Ausgangsmolekül erhältlich ist durch ein- oder mehrfache konservative Aminosäuresubstitution, wobei die Protease aufweist:
i) Aminosäuresubstitutionen S3T, V4I, R99E und V199I an den Positionen, die den Positionen 3, 4, 99 und 199 entsprechen, und
ii) an mindestens einer der Positionen, die den Positionen 9, 21, 42, 44, 105, 112, 113, 131, 137, 139, 141, 145, 159, 168, 176, 177, 182, 193, 198, 204, 205, 206, 210, 212, 230, 234, 250, 253, 255, 259 oder 267 entsprechen, mindestens eine Aminosäuresubstitution; oder
(b) sie aus einer Protease nach einem der Ansprüche 1 bis 3 als Ausgangsmolekül erhältlich ist durch Fragmentierung, Deletions-, Insertions- oder Substitutionsmutagenese und eine Aminosäuresequenz umfasst, die über eine Länge von mindestens 190, 200, 210, 220, 230, 240, 250, 260, 261, 262, 263, 264, 265, 266, 267, 268 oder 269 zusammenhängenden Aminosäuren mit dem Ausgangsmolekül übereinstimmt, wobei die Protease aufweist:
i) Aminosäuresubstitutionen S3T, V4I, R99E und V199I an den Positionen, die den Positionen 3, 4, 99 und 199 entsprechen, und
ii) an mindestens einer der Positionen, die den Positionen 9, 21, 42, 44, 105, 112, 113, 131, 137, 139, 141, 145, 159, 168, 176, 177, 182, 193, 198, 204, 205, 206, 210, 212, 230, 234, 250, 253, 255, 259 oder 267 entsprechen, mindestens eine Aminosäuresubstitution.

5. Verfahren zur Herstellung einer Protease, umfassend das Einbringen
i) von Aminosäuresubstitutionen S3T, V4I, R99E und V199I an den Positionen, die bezogen auf die Nummerierung gemäß SEQ ID NO:1 den Positionen 3, 4, 99 und 199 entsprechen, und
ii) mindestens einer Aminosäuresubstitution an mindestens einer der Positionen, die bezogen auf die Nummerierung gemäß SEQ ID NO:1 den Positionen 9, 21, 42, 44, 105, 112, 113, 131, 137, 139, 141, 145, 159, 168, 176, 177, 182, 193, 198, 204, 205, 206, 210, 212, 230, 234, 250, 253, 255, 259 oder 267 entsprechen,
in ein Ausgangsmolekül, das eine Aminosäuresequenz aufweist, die mindestens 70% Sequenzidentität mit der in SEQ ID NO:1 angegebenen Aminosäuresequenz über deren Gesamtlänge aufweist.

6. Verfahren nach Anspruch 5, ferner umfassend einen oder mehreren der folgenden Verfahrensschritte:
(a) Einbringen einer ein- oder mehrfachen konservativen Aminosäuresubstitution, wobei die Protease aufweist:
i) Aminosäuresubstitutionen S3T, V4I, R99E und V199I an den Positionen, die den Positionen 3, 4, 99 und 199 entsprechen, und
ii) an mindestens einer der Positionen, die den Positionen 9, 21, 42, 44, 105, 112, 113, 131, 137, 139, 141, 145, 159, 168, 176, 177, 182, 193, 198, 204, 205, 206, 210, 212, 230, 234, 250, 253, 255, 259 oder 267 entsprechen, mindestens eine Aminosäuresubstitution;
(b) Veränderung der Aminosäuresequenz durch Fragmentierung, Deletions-, Insertions- oder Substitutionsmutagenese derart, dass die Protease eine Aminosäuresequenz umfasst, die über eine Länge von mindestens 190, 200, 210, 220, 230, 240, 250, 260, 261, 262, 263, 264, 265, 266, 267, 268 oder 269 zusammenhängenden Aminosäuren mit dem Ausgangsmolekül übereinstimmt, wobei die Protease aufweist:
i) Aminosäuresubstitutionen S3T, V4I, R99E und V199I an den Positionen, die den Positionen 3, 4, 99 und 199 entsprechen, und
ii) an mindestens einer der Positionen, die den Positionen 9, 21, 42, 44, 105, 112, 113, 131, 137, 139, 141, 145, 159, 168, 176, 177, 182, 193, 198, 204, 205, 206, 210, 212, 230, 234, 250, 253, 255, 259 oder 267 entsprechen, mindestens eine Aminosäuresubstitution.

7. Nukleinsäure codierend für eine Protease nach einem der Ansprüche 1 bis 4 oder codierend für eine nach einem Verfahren der Ansprüche 5 bis 6 erhaltene Protease.

8. Vektor enthaltend eine Nukleinsäure nach Anspruch 7, insbesondere ein Klonierungsvektor oder ein Expressionsvektor.

9. Nicht-menschliche Wirtszelle, die eine Nukleinsäure nach Anspruch 7 oder einen Vektor nach Anspruch 8 beinhaltet, oder die eine Protease nach einem der Ansprüche 1 bis 4 beinhaltet, oder die eine nach einem Verfahren der Ansprüche 5 bis 6 erhaltene Protease beinhaltet, insbesondere eine, die die Protease in das die Wirtszelle umgebende Medium sezerniert.

10. Verfahren zur Herstellung einer Protease umfassend
a) Kultivieren einer Wirtszelle gemäß Anspruch 9 und
b) Isolieren der Protease aus dem Kulturmedium oder aus der Wirtszelle.

11. Mittel, insbesondere ein Wasch- oder Reinigungsmittel, insbesondere ein flüssiges Waschmittel oder ein Handgeschirrspülmittel, **dadurch gekennzeichnet, dass** es mindestens eine Protease nach einem der Ansprüche 1 bis 4 oder eine nach einem Verfahren der Ansprüche 5 bis 6 erhaltene Protease enthält.

12. Verfahren zur Reinigung von Textilien oder harten Oberflächen, **dadurch gekennzeichnet, dass** in mindestens einem Verfahrensschritt ein Mittel nach Anspruch 11 angewendet wird.

13. Verwendung einer Protease nach einem der Ansprüche 1 bis 4 oder eine nach einem Verfahren der Ansprüche 5 bis 6 erhaltene Protease in einem Wasch- oder Reinigungsmittel zur Entfernung von peptid- oder proteinhaltigen Anschmutzungen.

## Claims

1. Protease comprising an amino acid sequence having at least 70% sequence identity to the amino acid sequence set forth in SEQ ID NO:1 over the total length thereof and which, based on the numbering according to SEQ ID NO:1, has at
i) the positions corresponding to positions 3, 4, 99 and 199 the amino acid substitutions S3T, V4I, R99E and V199I, and
ii) at least one of the positions corresponding to positions 9, 21, 42, 44, 105, 112, 113, 131, 137, 139, 141, 145, 159, 168, 176, 177, 182, 193, 198, 204, 205, 206, 210, 212, 230, 234, 250, 253, 255, 259 or 267 at least one amino acid substitution.

2. Protease according to claim 1, wherein
the at least one amino acid substitution at at least one of the positions corresponding to positions 9, 21, 42, 44, 105, 112, 113, 131, 137, 139, 141, 145, 159, 168, 176, 177, 182, 193, 198, 204, 205, 206, 210, 212, 230, 234, 250, 253, 255, 259 or 267 is selected from the group consisting of 9C, 21F, 21W, 42S, 42H, 44S, 105V, 112V, 113A, 131D, 137I, 139R, 141S, 145I, 159L, 168V, 176E, 177D, 182C, 193M, 198D, 204L, 205D, 206A, 210C, 212D, 230E, 234A, 250N, 253C, 255Y, 259C and 267S.

3. Protease according to any one of claims 1 to 2, wherein the protease comprises, in each case based on numbering according to SEQ ID NO:1:
i) the amino acid substitutions S3T + V4I + R99E + V199I and
ii) any one of the following amino acid substitution combinations: (i) A267S + N255Y; (ii) N198D + S234A + L21F; (iii) N212D; (iv) G205D; (v) S182C; (vi) R44S + S234A; (vii) S253C; (viii) I105V;(ix) N177D; (x) V193M; (xi) S9C; (xii) N198D; (xiii) S259C; (xiv) I159L + Q230E; (xv) P204L; (xvi) L21F; (xvii) N42H; (xviii) S139R + S250N; (xix) N42S; (xx) V137I; (xxi) V145I; (xxii) S206A; (xxiii) G113A + L21W; (xxiv) A112V; (xxv) A168V; (xxvi) Q176E + A131D; (xxvii) T141S + S210C.

4. Protease, **characterized in that**
(a) it is obtainable from a protease according to any one of claims 1 to 3 as a starting molecule by single or multiple conservative amino acid substitution, wherein the protease comprises:
i) amino acid substitutions S3T, V4I, R99E and V199I at positions corresponding to positions 3, 4, 99 and 199, and
ii) at least one amino acid substitution at at least one of the positions corresponding to positions 9, 21, 42, 44, 105, 112, 113, 131, 137, 139, 141, 145, 159, 168, 176, 177, 182, 193, 198, 204, 205, 206, 210, 212, 230, 234, 250, 253, 255, 259 or 267, or
(b) it is obtainable from a protease according to any one of claims 1 to 3 as a starting molecule by fragmentation, deletion, insertion or substitution mutagenesis and comprises an amino acid sequence which matches the starting molecule over a length of at least 190, 200, 210, 220, 230, 240, 250, 260, 261, 262, 263, 264, 265, 266, 267, 268 or 269 contiguous amino acids, wherein the protease has:
i) amino acid substitutions S3T, V4I, R99E and V199I at positions corresponding to positions 3, 4, 99 and 199, and
ii) at least one amino acid substitution at at least one of the positions corresponding to positions 9, 21, 42, 44, 105, 112, 113, 131, 137, 139, 141, 145, 159, 168, 176, 177, 182, 193, 198, 204, 205, 206, 210, 212, 230, 234, 250, 253, 255, 259 or 267.

5. Method for producing a protease, comprising introducing
i) of amino acid substitutions S3T, V4I, R99E and V199I at the positions corresponding to positions 3, 4, 99 and 199, based on numbering according to SEQ ID NO:1, and
ii) at least one amino acid substitution at at least one of the positions corresponding to positions 9, 21, 42, 44, 105, 112, 113, 131, 137, 139, 141, 145, 159, 168, 176, 177, 182, 193, 198, 204, 205, 206, 210, 212, 230, 234, 250, 253, 255, 259 or267, based on numbering according to SEQ ID NO:1,
into a starting molecule having an amino acid sequence that has at least 70% sequence identity to the amino acid sequence set forth in SEQ ID NO:1 over the total length thereof.

6. Method according to claim 5, further comprising one or more of the following method steps:
(a) introducing a single or multiple conservative amino acid substitution, wherein the protease has:
i) amino acid substitutions S3T, V4I, R99E and V199I at positions corresponding to positions 3, 4, 99 and 199, and
ii) at least one amino acid substitution at at least one of the positions corresponding to positions 9, 21, 42, 44, 105, 112, 113, 131, 137, 139, 141, 145, 159, 168, 176, 177, 182, 193, 198, 204, 205, 206, 210, 212, 230, 234, 250, 253, 255, 259 or 267;
(b) altering the amino acid sequence by fragmentation, deletion, insertion or substitution mutagenesis such that the protease comprises an amino acid sequence that matches the parent molecule over a length of at least 190, 200, 210, 220, 230, 240, 250, 260, 261, 262, 263, 264, 265, 266, 267, 268 or 269 contiguous amino acids, wherein the protease has:
i) amino acid substitutions S3T, V4I, R99E and V199I at positions corresponding to positions 3, 4, 99 and 199, and
ii) at least one amino acid substitution at at least one of the positions corresponding to positions 9, 21, 42, 44, 105, 112, 113, 131, 137, 139, 141, 145, 159, 168, 176, 177, 182, 193, 198, 204, 205, 206, 210, 212, 230, 234, 250, 253, 255, 259 or 267.

7. A nucleic acid encoding a protease according to any one of claims 1 to 4 or encoding a protease obtained by a method according to any one of claims 5 to 6.

8. Vector containing a nucleic acid according to claim 7, in particular a cloning vector or an expression vector.

9. A non-human host cell comprising a nucleic acid according to claim 7 or a vector according to claim 8 or comprising a protease according to any one of claims 1 to 4, or comprising a protease obtained by a method according to any one of claims 5 to 6, in particular one that secretes the protease into the medium surrounding the host cell.

10. Method for producing a protease comprising
(a) culturing a host cell according to claim 9; and
(b) isolating the protease from the culture medium or from the host cell.

11. Agent, in particular a detergent or cleaning agent, in particular a liquid detergent or a hand dishwashing detergent, **characterized in that** it contains at least one protease according to any one of claims 1 to 4 or a protease obtained by a method according to any one of claims 5 to 6.

12. Method for cleaning textiles or hard surfaces, **characterized in that** in at least one method step an agent according to claim 11 is applied.

13. Use of a protease according to any one of claims 1 to 4 or a protease obtained by a method according to any one of claims 5 to 6 in a washing or cleaning composition for removing peptide- or protein-containing soils.

## Revendications

1. Protéase comprenant une séquence d'acides aminés qui présente au moins 70 % d'identité de séquence avec la séquence d'acides aminés indiquée dans la SEQ ID NO:1 sur sa longueur totale et qui, par rapport à la numérotation selon la SEQ ID NO:1, se situe au niveau de la séquence d'acides aminés indiquée dans la SEQ ID NO:1
i) les positions correspondant aux positions 3, 4, 99 et 199 ont les substitutions d'acides aminés S3T, V4I, R99E et V199I, et
ii) au moins une des positions correspondant aux positions 9, 21, 42, 44, 105, 112, 113, 131, 137, 139, 141, 145, 159, 168, 176, 177, 182, 193, 198, 204, 205, 206, 210, 212, 230, 234, 250, 253, 255, 259 ou 267 comporte au moins une substitution d'acide aminé.

2. Protéase selon la revendication 1, dans laquelle
la au moins une substitution d'acide aminé à au moins une des positions correspondant aux positions 9, 21, 42, 44, 105, 112, 113, 131, 137, 139, 141, 145, 159, 168, 176, 177, 182, 193, 198, 204, 205, 206, 210, 212, 230, 234, 250, 253, 255, 259 ou 267, est choisi dans le groupe constitué par 9C, 21F, 21W, 42S, 42H, 44S, 105V, 112V, 113A, 131D, 137I, 139R, 141S, 145I, 159L, 168V, 176E, 177D, 182C, 193M, 198D, 204L, 205D, 206A, 210C, 212D, 230E, 234A, 250N, 253C, 255Y, 259C et 267S.

3. Protéase selon l'une quelconque des revendications 1 à 2, dans laquelle la protéase comprend, dans chaque cas par rapport à la numérotation selon SEQ ID NO:1:
i) les substitutions d'acides aminés S3T + V4I + R99E + V199I et
ii) l'une des combinaisons de substitution d'acides aminés suivantes : (i) A267S + N255Y ; (ii) N198D + S234A + L21F ; (iii) N212D ; (iv) G205D ; (v) S182C ; (vi) R44S + S234A ; (vii) S253C ; (viii) I105V ; (ix) N177D ; (x) V193M ; (xi) S9C ; (xii) N198D ; (xiii) S259C ; (xiv) I159L + Q230E ; (xv) P204L ; (xvi) L21F ; (xvii) N42H ; (xviii) S139R + S250N ; (xix) N42S ; (xx) V137I ; (xxi) V145I ; (xxii) S206A ; (xxiii) G113A + L21W ; (xxiv) A112V ; (xxv) A168V ; (xxvi) Q176E + A131D; (xxvii) T141S + S210C.

4. Protéase, **caractérisée en ce que**
(a) elle peut être obtenue à partir d'une protéase selon l'une quelconque des revendications 1 à 3 en tant que molécule de départ par une ou plusieurs substitutions conservatrices d'acides aminés, la protéase comprenant:
i) des substitutions d'acides aminés S3T, V4I, R99E et V199I aux positions correspondant aux positions 3, 4, 99 et 199, et
ii) à au moins une des positions correspondant aux positions 9, 21, 42, 44, 105, 112, 113, 131, 137, 139, 141, 145, 159, 168, 176, 177, 182, 193, 198, 204, 205, 206, 210, 212, 230, 234, 250, 253, 255, 259 ou 267, au moins une substitution d'acide aminé; ou
(b) elle peut être obtenue à partir d'une protéase selon l'une quelconque des revendications 1 à 3 en tant que molécule de départ par fragmentation, mutagenèse par délétion, mutagenèse par insertion ou mutagenèse par substitution et comprend une séquence d'acides aminés qui correspond à la molécule de départ sur une longueur d'au moins 190, 200, 210, 220, 230, 240, 250, 260, 261, 262, 263, 264, 265, 266, 267, 268 ou 269 acides aminés contigus, la protéase ayant:
i) des substitutions d'acides aminés S3T, V4I, R99E et V199I aux positions correspondant aux positions 3, 4, 99 et 199, et
ii) à au moins une des positions correspondant aux positions 9, 21, 42, 44, 105, 112, 113, 131, 137, 139, 141, 145, 159, 168, 176, 177, 182, 193, 198, 204, 205, 206, 210, 212, 230, 234, 250, 253, 255, 259 ou 267, au moins une substitution d'acide aminé.

5. Procédé de production d'une protéase, comprenant l'introduction
i) des substitutions d'acides aminés S3T, V4I, R99E et V199I aux positions correspondant aux positions 3, 4, 99 et 199 par rapport à la numérotation de SEQ ID NO:1, et
ii) d'au moins une substitution d'acide aminé en au moins une des positions qui, par rapport à la numérotation selon SEQ ID NO:1, correspondent aux positions 9, 21, 42, 44, 105, 112, 113, 131, 137, 139, 141, 145, 159, 168, 176, 177, 182, 193, 198, 204, 205, 206, 210, 212, 230, 234, 250, 253, 255, 259 ou 267,
en une molécule de départ ayant une séquence d'acides aminés qui présente une identité de séquence d'au moins 70 % avec la séquence d'acides aminés indiquée dans SEQ ID NO:1 sur sa longueur totale.

6. Procédé selon la revendication 5, comprenant en outre une ou plusieurs des étapes de procédé suivantes:
(a) introduction d'une substitution conservatrice d'acides aminés à une ou plusieurs reprises, la protéase comprenant:
i) des substitutions d'acides aminés S3T, V4I, R99E et V199I aux positions correspondant aux positions 3, 4, 99 et 199, et
ii) à au moins une des positions correspondant aux positions 9, 21, 42, 44, 105, 112, 113, 131, 137, 139, 141, 145, 159, 168, 176, 177, 182, 193, 198, 204, 205, 206, 210, 212, 230, 234, 250, 253, 255, 259 ou 267, au moins une substitution d'acide aminé;
(b) modification de la séquence d'acides aminés par fragmentation, mutagenèse par délétion, mutagenèse par insertion ou mutagénèse par substitution de telle sorte que la protéase comprend une séquence d'acides aminés qui correspond à la molécule de départ sur une longueur d'au moins 190, 200, 210, 220, 230, 240, 250, 260, 261, 262, 263, 264, 265, 266, 267, 268 ou 269 acides aminés contigus, dans laquelle la protéase a:
i) des substitutions d'acides aminés S3T, V4I, R99E et V199I aux positions correspondant aux positions 3, 4, 99 et 199, et
ii) à au moins une des positions correspondant aux positions 9, 21, 42, 44, 105, 112, 113, 131, 137, 139, 141, 145, 159, 168, 176, 177, 182, 193, 198, 204, 205, 206, 210, 212, 230, 234, 250, 253, 255, 259 ou 267, au moins une substitution d'acide aminé.

7. Acide nucléique codant pour une protéase selon l'une quelconque des revendications 1 à 4 ou codant pour une protéase obtenue par un procédé selon les revendications 5 à 6.

8. Vecteur contenant un acide nucléique selon la revendication 7, en particulier un vecteur de clonage ou un vecteur d'expression.

9. Cellule hôte non humaine comprenant un acide nucléique selon la revendication 7 ou un vecteur selon la revendication 8, ou comprenant une protéase selon l'une quelconque des revendications 1 à 4, ou comprenant une protéase obtenue par un procédé selon les revendications 5 à 6, notamment une qui sécrète la protéase dans le milieu entourant la cellule hôte.

10. Procédé de production d'une protéase comprenant
a) cultiver une cellule hôte selon la revendication 9 et
b) isoler la protéase du milieu de culture ou de la cellule hôte.

11. Produit, en particulier un produit de lavage ou de nettoyage, notamment un produit de lavage liquide ou un produit de lavage de la vaisselle à la main, **caractérisé en ce qu'**il contient au moins une protéase selon l'une des revendications 1 à 4 ou une protéase obtenue selon un procédé des revendications 5 à 6.

12. Procédé de nettoyage de textiles ou de surfaces dures, **caractérisé en ce que**, dans au moins une étape du procédé, on utilise un produit selon la revendication 11.

13. Utilisation d'une protéase selon l'une des revendications 1 à 4 ou d'une protéase obtenue selon un procédé des revendications 5 à 6 dans un produit de lavage ou de nettoyage pour éliminer des salissures contenant des peptides ou des protéines.
